# EUROPEAN PATENT SPECIFICATION

(11) **EP 1 802 980 B1**
(45) Date of publication and mention of the grant of the patent: **05.12.2012**
(21) Application number: 05789775.3
(22) Date of filing: 07.10.2005
(51) Int. Cl.: G01N 33/68

(54) **METHODS FOR ANTIBODY LIBRARY SCREENING**
METHODEN ZUM SCREENING VON ANTIKOERPER BIBLIOTHEKEN
PROCEDE DE CRIBLAGE D'UNE BANQUE D'ANTICORPS

(30) Priority: 08.10.2004 GB 0422433; 08.10.2004 GB 0422431
(43) Date of publication of application: 04.07.2007
(73) Proprietor: Affitech Research AS, 0349 Oslo (NO)
(72) Inventor: STASSAR, Marike, Josée, Janneke, Gertrud, 1353 Baerums Verk (NO); REIERSEN, Herald, N-1412 Sofiemyr (NO)
(74) Representative: Rigby, Barbara Nicole
(86) International application number: PCT/GB2005/003865
(87) International publication number: WO 2006/038021

(56) References cited:
- WO-A-2004/056995
- US-A1- 2003 040 605
- WATKINS JEFFRY D ET AL: "Discovery of human antibodies to cell surface antigens by capture lift screening of phage-expressed antibody libraries" ANALYTICAL BIOCHEMISTRY, vol. 256, no. 2, 15 February 1998 (1998-02-15), pages 169-177, XP002357971 ISSN: 0003-2697
- STASSAR M J J G ET AL: "CBAS: A new method for the identification of single chain antibodies specific for cell surface antigens" HUMAN ANTIBODIES, vol. 13, no. 1-2, 2004, pages 35-36, XP009058189 & 11TH INTERNATIONAL CONFERENCE ON HUMAN ANTIBODIES AND HYBRIDOMAS; DUBLIN, IRELAND; OCTOBER 06-08, 2004 ISSN: 1093-2607

## Description

This invention relates to a new method to isolate affinity library members via their ligand. In particular, the invention relates to a method of screening a library of molecules (affinity library members) to identify or select one or more members thereof which are candidate binding partners for one or more ligands, in particular candidate binding partners for ligands on the surface of cells as set out in claim 1. A preferred embodiment of the present invention provides an improved method of library screening which combines a solid phase selection step and the use of PCR for the detection of the presence of ligand.

Screening libraries has now become a routine procedure in drug discovery and biotechnology (Emili, A. Q, and Cagney, G. (2000) Nat. Biotechnol. 18, 393-397; Li, M. (2000) Nature Biotechnol. 18, 1251-1256). One major application is the identification of proteins of clinical interest (Burton, D. R. (2002) Nature Rev. Immunol. 2, 706-713). To this end, a library containing several million different member molecules (typically protein or peptide members) are screened for affinities against a ligand of interest (Winter, G. P. et al, WO 90/05144; McCafferty, J. et al., WO 92/01047; Breitling, F. et al., WO 93/01288). This ligand may itself be a cell (or a cell surface molecule), a molecule or a library of molecules, for example living eukaryotic cells, proteins, peptide or small chemical compounds.

Despite the fact that some screening, methods are described, new or improved methods with respect to speed, sensitivity, throughput and reproducibility have to be developed to be able to generate promising drug candidates, especially to more challenging ligands. In addition, cost efficiency is a major factor when evaluating screening methods. Today, there are several selection systems available for direct identification of members from *in vivo* and *in vitro* libraries. The vast majority of these which are designed to identify molecules of pharmaceutical interest share a common feature: the linkage of the phenotype of each individual library member with its specific genotype. This is either via phage-coat proteins, cell-membranes or ribonucleic acid-protein complexes. The linkage, a prerequisite to the function of those screening systems, may be constructed differently for library display on for example phages (Winter, G. P. et al, WO 90/05144; McCafferty, J. et al., WO 92/01047; Breitling, F. et al., WO 93/01288; Ladner, R. C., et al., WO90/02809; Markland, W. et al., WO 92/15679; Harrison, J. L. et al (1996) Meth. Enzymol. 267, 83-109), bacteria (Samuelson, P.et al (2002) J. Biotechnol. 96, 129-154), yeast (Wittrup, K. D. et al., WO 99/36569; Wittrup, K. D. (2001) Curr. Opin. Biotechnol. 12, 395-3 99; Lee, S. Y. et al (2003) Trends in Biotechnol. 21, 45-52), or with ribosome display (Kawasaki, G., WO91/05058; Mattheakis, L. C. et al, WO95/11922; Plückthun, A. et al., WO 98/48008; Schaffitzel, C. et al (1999) J. Immunol. Meth. 231, 119-135; Coia, G. et al. (2001) J. Immunol. Meth. 254, 191-197), covalent display (Szostak, J. W. et al. WO 98/31700; Szostak, J. W. et al. WO 00/47775; Lindqvist, B. H. et al. WO 98/37186; Amstutz, P. et al. (2001) Curr. Opin. Biotechnol. 12, 400-405; Wilson, D. S. et al. (2001) Proc. Natl. Acad. Sci. USA 98,3750-3755), CIS-display (McGregor, D. et al. WO 04/022746; Odegrip, R. et al. (2004) Proc. Natl. Acad. Sci. USA 101, 2806-2810) and bacterial two-hybrid systems (Chien et al. (1991) Proc. Natl. Acad. Sci. USA 88, 9578). The idea behind all these methods however is to translate and display the affinity molecule using a system that makes it possible to enrich, isolate and identify the library member.

To isolate candidates, the library of interest is exposed to a ligand, which in antibody discovery is an antigen. The ligand is typically immobilised on a solid support such as e.g. in WO2004/056995. This support may often be the plastic surfaces of beads, microtitre plates or immunotubes. Alternatively, the interaction may take place in solution on tagged ligand targets (e.g. by biotin). The procedure involves several washing steps to remove unspecific and non-reactive library members (panning). To purify complexes in solution, they have to be captured by either immobilisation or by a centrifugation step. One traditional method is to capture the affinity member on a soluble biotinylated ligand, and to immobilise the affinity complex(affinity member and ligand) on streptavidin beads. By using beads as a solid support, the alternatives are many, for example magnetic beads (e.g. from Bangs Laboratories, Polysciences inc., Dynal Biotech, Miltenyi Biotech or Quantum Magnetic), non-magnetic beads (e.g. Pierce and Upstate technology), monodisperse beads (e.g. Dynal Biotech and Microparticle Gmbh) and polydisperse beads (e.g. Chemagen). The use of magnetic beads has been described exhaustingly in literature, generally on how to use beads to capture protein, DNA, viruses, bacteria and eukaryotic Cells (Uhlén, M, el al (1994) in Advances in Biomagnetic Separation, BioTechniques press, Westborough, MA). Uhlén *et al., supra,* refers to a direct positive isolation step. Also a negative election step is mentioned making it is possible to remove unwanted library members from a mixture.

All these examples are based on direct identification of the enriched library members by the fact that the displayed member molecule specifically recognizes the ligand. Typically, this includes a polyclonal ELISA, followed by cloning, DNA sequencing and expressing of single enriched library members in another system, (e.g. *E.coli*, yeast or with an *in vitro* translation kit) followed by further downstream analysis of the library member by for example BiaCore, ELISA and/or affinity measurements. In some cases the resulting pool of library members after panning may contain several candidate members with different affinities or with a larger pool of non-reactive members. To isolate a single specimen another screening platform may be applied to subscreen the enriched ensemble.

Filter screening procedures are commonly used for smaller chain-shuffled libraries (up to 10⁵ members). A small bacterial library is plated out on bioassay plates and individual clones are robot picked for high throughput screening (Walter, G. et al. (2002) Trends Mol. Medicine 8, 250-253). The bacterial library may be a tag-labelled scFv antibody expression library controlled by a lac promoter. The procedure involves a combination of specific gridding patterns of clones on a support filter and filter lift onto a capture filter. The capture filter may be-coated with the affinity ligand of interest (antigen) and placed on top of an IPTG bioassay plate for expression. The library members are identified by their unique gridding pattern of their tag on the capture filter using Western blotting techniques (Stacy, J. E., et al. (2003) J. Immunol. Meth. 283, 247-259). This method has even proven to be very well suited for direct screening of small patient libraries (Brekke, O. H., et al., WO 03/095491).

However, for all existing panning and screening methods currently mentioned in literature, the library members of interest are detected and isolated with a feature incorporated in the library itself. This might be a specific gridding pattern in case of filter screening method, the infectiveness of a phage for phage display, or a PCR signal of enriched DNA of the library member using *in vitro* displayed library screening technologies

There are however no methods available to identify library members via their ligands. All detection methods so far involve detecting the affinity library member. In contrast, the present invention provides a novel technique to indirectly identify the library member via detecting its ligand (or at least by detecting an entity with which the ligand is associated). This is of particular value in screening small libraries against complex ligands like live cells, although the applicability of the method is not limited to those applications.

Thus, at its most general the present invention provides a method of screening a library of molecules to identify and/or select one or more members thereof which are candidate binding partners for one or more ligands comprising:
a) contacting an expression library in solution with one or more ligands wherein said expression library is not attached to a solid phase;
b) capturing ligands which have become bound to one or more members of the expression library onto a solid phase, wherein said capture onto a solid phase step is facilitated by the expression library members and wherein said capture onto a solid phase involves the interaction of a capture molecule on the solid phase with a partner molecule or tag associated with the members of the expression library; and
c) detecting the presence of a ligand, thereby detecting the presence of one or more members of the expression library which are candidate binding partners for the ligand.

The library of molecules to be screened in accordance with the present invention can be any protein expression library, i.e. any known or newly derived peptide or polypeptide expression library. Examples of appropriate expression libraries are well known and described in the art (see the discussion above) and include display libraries such as phage display libraries (Winter et al., etc., *supra*)*,* bacteria (Samuelson et al., *supra*) or yeast display libraries (Wittrup et al., etc., *supra*)*,* covalent or non-covalent display libraries such as ribosome display libraries (for example as published in WO92/02536 of The Regents of the University of Colorado, WO93/03172 of University Research Corporation and WO91/05058 of Kawasaki), or PROfusion libraries (Phylos, Inc.), wherein the expressed protein is bound to the mRNA encoding it, or covalent or non-covalent display systems whereby the expressed protein is bound to the DNA encoding it (for example via a covalent linkage as in the systems described in WO98/37186 of Actinova Ltd or via cis acting proteins in the cis-display systems as described in WO04/022746), or bacterial two hybrid systems (Chien *et al., supra*). Alternative types of expression library can also readily be used such as bacterial expression libraries or other libraries wherein proteins are expressed and secreted as soluble fragments (soluble expression libraries). Chemical libraries can also be screened, e.g. synthetic peptide libraries, or chemical molecule libraries. Thus, at its broadest the present invention extends beyond the screening of protein expression libraries (biological libraries) to the screening of chemical libraries. Preferred libraries for use in the present invention are phage display libraries or bacterial expression libraries.

The proteins expressed by the expression library can be of any appropriate length providing that said length is sufficient to enable a protein to act (or potentially act) as a binding partner for a ligand. Thus, said proteins may be short linear peptides (e.g. of the order of 5-50 or 7-30 amino acids in length), or longer peptides or polypeptides which may be folded rather than linear. Thus, the proteins of the expression library may be encoded for by whole genes or fragments thereof, e.g. the nucleic acids encoding the expression library members may be a cDNA or mRNA library or fragments thereof, for example generated from a particular cell type or may be a genomic DNA library or fragments thereof.

Preferred expression libraries express polypeptide binding partners which are candidate ligands, receptors, enzymes, substrates, antigens, antibodies, etc., or fragments thereof, i.e. are affinity libraries, and especially preferred expression libraries express antibody molecules or antibody fragments, which can then be screened for candidates which bind to one or more known or unknown antigens (ligands). Said antibody expression libraries may express antibodies in any appropriate form and may comprise whole antibody molecules or antibody fragments such single chain antibodies (e.g. scFv), Fab, Fv, Fab'2, diabodies, bispecific antibodies, minibodies, heavy chains or light chains, triabodies, tetrabodies, cameloid antibodies, single domain antibodies, etc. A preferred format of antibody fragments are scFv fragments.

The antibody molecules or fragments may be of any Ig isotype, such as IgG, IgM or IgA and so forth, and the expression libraries may comprise antibodies of one or more of these subtypes. Many antibody libraries are known and described in the art and any of these, or a newly derived library, may be screened using the methods of the invention.

When an expression library is referred to herein, the term can be used to refer to such a library at the nucleic acid or protein level, i.e. before or after expression of the encoded proteins has taken place. Clearly however, such expression libraries must be present at the protein level in order for interaction with the ligands to take place. Thus, in order for the contacting step (a) to successfully occur, the expression libraries have to be present at the protein level (although initially they may be present at the nucleic acid level).

One of the main requirements for the expression libraries to be used in the methods of the invention is that they must be in solution when they are initially brought into contact with the ligands against which they are being screened. By "in solution" is meant that the expression libraries are not attached to a solid phase when they are initially brought into contact with the ligands against which they are being screened, i.e. the initial expression libraries are non-immobilized expression libraries.

Methods for constructing such expression libraries and nucleic acids encoding them are well known and described in the art and any known expression library or newly developed or constructed expression library can be used in this regard. For example such libraries may be comprised of Naturally occurring polypeptides or fragments thereof or may be wholly or partially random or synthetic. For example, in the case of antibody expression libraries, the libraries may be derived by cloning nucleic acids from a naive population of lymphocytes from a healthy donor or from an enriched population of lymphocytes, e.g. lymphocytes derived from a patient which has been exposed to antigen or immunized with a vaccine or for example tumour cells (e.g. as described in WO03/095491 of Affitech AS), or from lymphocyte populations which have been enriched by panning on particular antigens.

The-expression libraries and in particular the antibody expression libraries may be derived from any appropriate source, preferably from a mammalian source, more preferably a human source. Chimeric expression libraries or humanized expression libraries may also be used. The libraries may also be oreated by choosing a naturally occurring scaffold and including randomized sequences at appropriate places. Alternatively, the scaffold may be based on one or more consensus sequences derived from a variety of naturally occurring frameworks.

Generally, the techniques used to prepare the library constructs will be based on known genetic engineering techniques. In this regard, nucleic acid sequences encoding the actual protein or peptide or fragment thereof which is to be expressed in the expression library and which generally vary between different library members, thereby providing the library diversity, are incorporated into expression vectors appropriate for the type of expression system to be used. Appropriate expression vectors for use in phage display, covalent and non-covalent display, bacterial expression etc., are well known and described in the art (see for example the references referred to in the above discussion of appropriate types ofexpression library for use in the present invention). If phage display is the expression library of choice then either phage or phagemid vectors may be used, although phagemid vectors are preferred. In embodiments of the invention where the expression libraries are libraries of antibody fragments, an appropriate design of expression vector to enable the expression of antibody fragments in the desired format would be well within the normal practice of a person skilled in the art.

Once generated the nucleic acid molecules encoding different library members (i.e. encoding the proteins or peptides which vary between the library members, i.e. the expression peptides) can also be further diversified using standard techniques, for example by mutation involving the addition, deletion and/or substitution of one or more nucleotides in a controlled (e.g. site directed mutagenesis) or random manner, or by domain swapping, cassette mutagenesis, chain shuffling etc. Synthetic nucleotides may be used in the generation of the diverse nucleic acid sequences. Thus, all or part of the nucleic acids encoding the expression peptides can be synthesized chemically or be derived from various organisms or cell types.

In addition, the expression libraries for use in the methods of the present invention may already have been educed in complexity by for example carrying out one or more rounds of panning or selection against the target ligand or a non-target ligand (negative panning). Such a reduced expression library may optionally be subjected to further diversification, e.g. using the methods discussed above, before screening using the methods of the present invention.

The expression library constructs may optionally additionally contain other appropriate components, for example origins of replication, inducible or non-inducible promoters for initiating transcription, enhancers, antibiotic resistance genes and markers, general tags or reporter molecules, primer binding sites to enable amplification of the constructs by e.g. PCR, or other desirable sequence elements. Appropriate sources and positioning of such additional components within the library constructs so that they perform their desired function would be well within the normal practice of a skilled person in the art.

The inclusion of general tags or markers in the library constructs and in the expressed library members is particularly important in the expression libraries for use in the present invention and such tags are used to facilitate the binding of library members to a solid phase in the capture step of the method of the invention, i.e. are used to facilitate the capture step. Any appropriate tag may be used in this regard providing it can facilitate binding of the library members to a solid phase. However, conveniently such tags are affinity molecules which can facilitate binding to a solid phase by binding to a partner affinity molecule immobilized directly or indirectly onto the solid phase. Exemplary tags may be c-myc tags (which can for example be captured via an anti c-myc antibody) or His tags (which can for example be captured via a Nickel surface) or biotin (which can for example be captured via streptavidin molecules), or a phage surface protein such as gp8, (which can for example be captured via an anti gp8 antibody), or antigen peptide tags such as FLAG or HA which may be recognized by an antibody. Such tags or markers may conveniently also be directly or indirectly detectable. Such tags or markers are typically general tags or markers which are present in all the library constructs (i.e. each of the members of the expression library have the same tag or marker) and can be used to capture the library members onto a solid phase. Such tags or markers are not required for the detection step of the methods of the invention (as detection is carried out via the detection of the ligand rather than the library member, see in more detail below). However, if desired such tags can be used to detect the presence of library members. Their pres ence can prove extremely useful in determining the affinity of a ligand for a library member.

Once the appropriate expression library constructs have been obtained at the nucleic acid level, these can then be expressed for screening and for use in step (a) of the method in appropriate expression systems, depending on the nature of the library constructs, e.g. depending on whether phage display, *in vitro* display or bacterial expression is the system of choice. Appropriate conditions and methods of expression are well known and described in the art.

The term "ligand" as used herein refers to any entity (sometimes referred to as a "target ligand") or molecule of interest to which it is desired to identify a proteinaceous binding partner from an expression library. Such ligands thus have to be capable of binding or otherwise interacting with members of the protein expression library being screened and can be proteins/peptides, glycopeptides, carbohydrates, lipids, glycolipids, small-chemical molecules, etc.

Such ligands may be cell surface molecules attached to or being components of whole cells, or may be for example free, naked, isolated or purified molecules. A single ligand or multiple ligands (e.g. a library of ligands) may be present. Preferred ligands are proteins or peptides, e.g. antigens. More preferred ligands are cell surface molecules (i.e. molecules present *in situ* on the surfaces of cells), and in particular cell surface proteins or peptides, e.g. cell surface antigens. Such ligands may be known or unknown molecules for which it is desired to identify candidate binding partners. For use in the methods of the present invention, such ligands are generally attached to an appropriate moiety in order to facilitate the detection step, and/or to facilitate easy manipulation, e.g. washing steps etc., of the ligand. If the ligand is a cell surface molecule then the cell itself provides such a moiety. If however, the ligand is a naked, isolated or purified molecule then such molecules are conveniently attached to a solid phase to provide such a moiety.

The term "cell" as used herein refers to any type of biological particle and includes bacteria, viruses and eukaryotic cells (including lower eukaryotic cells such as yeast cells and higher eukaryotic cells such as mammalian cells, in particular human cells). The cells may be naturally occurring cells or a mixture of cells (e.g. cells derived from a biopsy-or some other sample from a mammalian subject), or cell lines (including immortalized cells and genetically engineered cell lines, for example cells transformed or transfected with a nucleic acid encoding a particular ligand or a plurality of cells transformed or transfected with a library of ligands, e.g. a cDNA library), etc. Cells which overexpress said ligand can also be used. Fragments of cells are also included within the ambit of this term, e.g. membrane fractions, cell wall fractions, cell wall proteins, membrane proteins, etc.

Preferred "cells" for use in the present invention are eukaryotic cells or membrane fractions thereof. As alluded to above, such eukaryotic cells (or indeed other cell types) may have been transfected with a. library of molecules (ligands) which are then expressed on the surface of the cells and can be subjected to the methods of the invention in order to identify candidate binding partners for the expressed molecules. In this way the invention provides methods of screening libraries against libraries, which is particularly advantageous. Preferred libraries of ligand are libraries produced from disease associated entities, e.g. disease associated cells or pathological agents such as viruses or bacteria. A particularly preferred library of ligand is a tumour cell library (e.g. a cDNA library) or a viral cell library (e.g. a cDNA library) to enable candidate binding partners to tumour or viral associated proteins to be identified. Appropriate eukaryotic cells (and other cell types) for transfection, such as for example COS cells, are well known and described in the art and any of these may be used.

Other preferred eukaryotic cells are those which are associated with a disease state such as for example cancer cells or cell lines, for example breast cancer or lung cancer cells or cell lines, or lymphocytes (e.g. peripheral blood lymphocytes) from a diseased patient, or cells infected with a virus and thereby presenting viral proteins on their surface. Such cells can be subjected to the screening methods of the invention in order to identify candidate binding partners for molecules/ligands, e.g. tumour associated molecules or infection associated molecules, expressed on the surface of the disease related cell.

The cells to be used in the methods of the invention can be derived from any appropriate source, for example the cells can be derived directly from a natural source, e.g. a mammalian subject or can be obtained from *in vitro* cultures, for example if transfected cells are to be used. If an *in vitro* culture is used then said cells may be cultured in suspension or as monolayers and may be used in the methods of the invention in a live or fixed state. It is generally preferred that live cells are used (and particularly preferably live eukaryotic cells) because then any ligand on the surface of the cell is present in its native form which means that the candidate binding partners selected by the method then recognize the target ligand in its native form, thereby increasing the chances that such candidate binding partners will be of therapeutic or diagnostic use. It is generally preferred that freshly isolated cells rather than frozen or stored cells are used in the methods of the invention. Fixed cells can however be used in the methods of the invention providing-such fixed cells are still in solution.

In embodiments of the invention where the ligand is a molecule attached to the surface of a solid phase then this molecule can be derived from any appropriate source, i.e. can be a naturally occurring protein, carbohydrate, lipid, glycolipid, etc., which has been isolated or purified from its natural surroundings, or it can be a recombinant or synthetic molecule, e.g. a recombinant protein or peptide or a synthesized chemical moiety. Isolated, purified or recombinant antigens are particularly preferred ligands in this regard.

Optionally and indeed preferably, such ligands are also associated with the nucleic acid encoding them, or are associated with some other form of molecular tag to enable subsequence identification of the ligand. This is particularly important when libraries of ligand are used, as then there preferably needs to be a convenient method to enable subsequent identification of the ligand which has bound to the candidate binding partner to take place. Methods of associating such ligands with the nucleic acid encoding them are well known and described in the art and can conveniently be achieved by for example using an *in vitro* display system such as for example ribosome display or covalent or non-covalent display techniques as discussed above, e.g. cis display etc. Alternatively, such association can be mediated by another form of affinity linkage, for example via a streptavidin-biotin linkage. In this regard, the nucleic acid of the ligand library members may be tagged with a biotin molecule and designed to encode a streptavidin molecule. When the protein of the library members are expressed, the streptavidin molecule then binds to the biotin molecule of the DNA and effects the linkage of the encoded protein to the nucleic acid encoding it.

Alternatively, such ligands can be labeled with DNA tags, such as for example oligonucleotides of different sizes or a specific sequence tag, which can subsequently be deconvoluted and used to identify the ligand, e.g. as described in Brenner et al., 2000, Proc. Natl. Acad. Sci. USA 97:1665-1670.

If the ligands are naked, isolated or purified molecules rather than molecules expressed in the context of a cell membrane, then these are generally attached to a solid phase before being screened using the methods of the invention. Appropriate solid phases are well known and described in the art and it is well within the normal practice of a skilled person to select the most appropriate solid phases for use in the methods of the invention. However, preferred solid phases to which the ligand is attached are particulate and non-magnetic, for example polymeric beads. An example of non-magnetic beads which can be used in the methods described herein are 5 µm glycidyl methacrylate microbeads (Bangs Laboratories, Carmel, IN). Thus, preferred ligands are polypeptides which have been attached to a particulate, non-magnetic, solid phase. When ligands are attached to a solid phase then the solid phase is generally used as a moiety to facilitate the detection step and/or a moiety to facilitate easy manipulation of the ligand, e.g. washing steps, etc. To distinguish these solid phases from the solid phases used to capture the ligand - expression library member complexes, these solid phases may be referred to as "ligand binding solid phases".

In such embodiments where the ligand is a naked, isolated or purified molecule, then these may be attached or immobilized to the surface of an appropriate solid phase by any appropriate method. Indeed, appropriate methods of binding polypeptides or other biomolecules to solid supports are well known and described in the art. Conveniently said attachment might take place using an affinity interaction, e.g. the polypeptides or other ligands are engineered to contain biotin molecules which can then be attached to streptavidin coated beads, preferably non-magnetic streptavidin beads, which are for example available commercially.

Whether the ligand is a cell surface molecule or a molecule of interest attached to an appropriate solid phase, the ligands contain or are associated with a reporter moiety to enable detection of the ligands in the method of the invention. Such reporter moieties are preferably nucleic acid molecules, more preferably DNA molecules, which can then be detected by nucleic acid based methods. A preferred method of detection is by PCR but any appropriate method can be used, for example hybridisation of a labelled probe. Alternatively, the ligand may be attached to a non-magnetic fluorescent bead, or to another population of beads of a distinguishable size that can be counted/detected in microscopy or by a Coulter Counter ZM (Coulter Electronics Ltd.). Thus, it can be seen that such reporter moieties can take a large variety of different forms providing detection of said reporter moieties is possible.

Such reporter moieties may be inherently associated with the ligands. For example, if the ligand is a cell surface molecule, then a preferred reporter moiety to be detected would be a DNA molecule, e.g. a gene (i.e. a reporter gene) which is present in the cells (e.g. is endogenous to the cells) on which the ligand is expressed/displayed. Appropriate and preferred reporter moieties are therefore moieties which are present in all cell types, e.g. a housekeeping gene such as β-actin.

Alternatively an exogenous reporter moiety may be associated with the ligand. For example, in the case of cells, an exogenous reporter moiety (e.g. a reporter gene) may be introduced into the cells by standard methods, e.g. transfection, including but not limited to lipofection, retroviral systems or electroporation.

If the ligand is a naked molecule, e.g. a naked polypeptide then this may be tagged or associated with an appropriate reporter moiety using methods well known and described in the art, for example if the ligand is associated with its encoding nucleic acid or another nucleic acid based tag, e.g. as described above, the reporter moiety can conveniently be provided within this nucleic acid molecule. If the ligand is a molecule, e.g. a polypeptide, attached to a particulate solid phase then said reporter moiety might be attached to the solid phase separately to the polypeptide, for example using similar methods to those described above. In particular, if streptavidin beads are used then the reporter moiety is preferably a biotinylated DNA fragment comprising a site or region which can facilitate detection of the reporter moiety, e.g. a primer site to enable PCR amplification of a fragment of the reporter gene or a probe binding site. Each bead can then carry a mixture of biotinylated reporter moiety and biotinylated polypeptide (or other ligand).

It can be seen therefore that such "association" between a reporter moiety and the ligand may comprise a direct interaction between the reporter moiety and the ligand, i.e. the reporter moiety and the ligand are part of the same molecule, e.g. the ligand protein and the reporter moiety are conjugated to one another as part of the same molecular entity. Alternatively, such "association" may be indirect, e.g. the reporter moiety is attached to or contained within a moiety to which the ligand is attached, e.g. is contained within the cell on which the ligand is expressed or displayed or immobilized onto the solid phase to which the ligand is attached.

Such reporter moieties are preferably general reporter moieties which are associated with all the ligands being displayed and can thus be used as a general reagent to detect the presence of any and all target ligands (and thereby the presence of a candidate binding partner).

"One or more" as used herein in connection with the term "ligand," refers to one or more distinct types of ligand, for example one or more different cell surface molecules or polypeptides. In other words the methods of the invention can be used to screen for candidate binding partners to one ligand or a plurality or library of ligands (e.g. can be used for library vs library screening which is particularly advantageous). If more than one target ligand is involved in the screening, these may be attached to or associated with the same or different moieties. For example, it can be seen that if the target ligands are cell surface molecules, then candidate binding partners for a number of different cell surface molecules are likely to be identified using the methods of the invention. Of course, if desired, this procedure can be biased towards the selection of candidate binding partners for a particular cell surface molecule, e.g. by selecting a cell which has an immunodominant target entity or was known or had been selected to overexpress the ligand of interest or had been engineered to overexpress such a ligand. Each ligand can of course be present in multiple copies in the screening reaction (indeed, this is preferred), e.g. if the ligand is a cell surface molecule then multiple copies of this are preferably present either on the same cell and/or on multiple cells of the same or different type.

The step of bringing the ligands into contact with the expression library or vice versa can be carried out in any appropriate way under conditions such that appropriate binding partners in the expression library can interact with or bind to the ligands which are present. Such conditions will generally vary depending on the nature of the expression library, the ligand and the moiety with which the ligand is associated. However, appropriate conditions to facilitate binding can be readily determined by a person skilled in the art. Such a "contacting" step will generally occur in an appropriate solution or aqueous medium. Thus, if the ligand is present on a cell which is cultured as a monolayer or cells which are obtained from a mammalian subject, e.g. from a mammalian organ or tissue, then these are generally harvested and resuspended in an aqueous medium by appropriate techniques before being contacted by the expression library.

Importantly, it has been found that the contacting step can be carried out in bacterial expression medium, for example L Broth, as well as in standard aqueous media such as PBS. This is important and surprising because it means that library members can be expressed in bacteria (e.g. by culturing them in a bacterial expression medium such as L Broth) and clones can be taken directly from the expression plate for use in the screening methods of the invention. Preferably such bacterial expression medium is made isotonic (e.g. to approximately 140 mM NaCl, (LB is usually approximately 171 mM NaCl)), for example with sterile water or other appropriate aqueous medium, before the ligands are added but after expression of the library members has been induced. This step of making the bacterial medium isotonic is especially preferred when the ligands are cell surface molecules on live cells rather than on beads.

Exemplary "contacting" conditions may comprise incubation on ice or at 4°C for between 30 minutes and 4 hours. However, these may be varied as appropriate depending on the nature of the ligands, expression libraries, etc. The expression library - ligand mixture may optionally and preferably be subjected to gentle rocking, mixing or rotation. In addition other appropriate reagents such as blocking agents to reduce non specific binding may be added. For example 1-4% BSA or other suitable blocking agent (e.g. milk) may be used. It will be appreciated however that the contacting conditions can be varied and adapted by a skilled person depending on the aim of the screening method. For example, if the incubation temperature is increased, for example to room temperature, this may increase the possibility of identifying binders to a different subset of ligands, e.g. binders to cell surface proteins which are readily internalized. Again such adaptations to the conditions are within the ambit of the skilled person.

The contacting step is preferably carried out in the wells of an assay plate or other appropriate reaction compartment or vessel. One type of library member (i.e. one member of the expression library) is conveniently present in each well or vessel. However, multiple types of library member (i.e. multiple members of the expression library) may also be present in each well or vessel (described in more detail below).

The size and complexity of the expression library to be used in the methods of the present invention (and therefore in the contacting step) may be varied as may the total number of copies of target ligands included in the contacting step. For example, the methods of the invention can be used to screen libraries with up to 500 000 different members, or libraries with 1x10⁶, 1x10⁸ or more members. Preferred libraries for screening have between 1 000 and 50 000 members, although the methods can clearly also be used for screening much smaller libraries, e.g. libraries with 50 to 1000, or 100 to 500 members.

Conveniently the number of copies of ligands can be varied by altering the number of moieties present with which the ligands are associated. For example, when the ligand is a cell surface molecule or a molecule attached to a solid support, then the number of target ligands can be adjusted by increasing or decreasing the number of cells or solid supports present in the contacting mixture. Again, the number of ligands required can readily be determined by trial and error, but for example when the ligand is a cell surface molecule 5 000 to 200 000 cells are conveniently used. Indeed it is an advantage of the screening method of the present invention that the screening method is sensitive enough to work with cell numbers as low as 5 000 or 1 000 cells, i.e. less than 5000 or 1000 cells (or even lower, e.g. the use of PCR as a method of detection should potentially enable the detection of a single cell). In this regard, particularly when screening with live cells derived from mammalian subjects, e.g. human patients, such cells are often rare resources and may not be available in large numbers. Thus, to be able to carry out screening on such low numbers of cells is an important advantage. The examples as attached hereto show that in a preferred embodiment of the invention where detection takes place by PCR and the expression library is an antibody library, only 40 pg of antibody is required to enable detection. This means that the methods of the present invention should be suited for isolating library members on ligand molecules which are expressed at a very low level, for example when less than 1000 molecules are expressed.

Once ligands have been contacted with the expression library under conditions such that the ligands can become bound to one or more members of the expression library, the ligands which have become bound in this way are then captured onto a solid phase to facilitate their isolation or removal or purification from the other components of the reaction mixture such as nonbound ligands. In other words, during the capture step only the ligands which are bound to an expression library member are captured onto the solid phase.

Preferred solid phases in this regard are particulate solid phases which facilitate easy manipulation and washing. Magnetic solid phases and in particular magnetic particles/beads are especially preferred. In order to distinguish these solid phases from the solid phases to which the ligands may be attached (as described above), such solid phases can be referred to as "capture solid phases". Suitable magnetic beads are available commercially from Dyno Specialty Polymers AS of Lillestrøm, Norway and Dynal Biotech ASA. Particular examples of magnetic beads which can be used in the methods described herein are the M-450, M-270 or M-280 beads from Dynal Biotech ASA, Norway.

In accordance with the present invention, capture onto a solid phase of ligand-library member complexes involves the interaction of a capture molecule on the solid phase with a partner molecule or tag associated with the members of the expression library. In this way only ligands which are bound to members of the expression library are captured onto the solid phase. All ligands not binding to members of the expression library can, if desired, be removed by one or more steps of washing the solid phase, or simply by separating the solid phase from the reaction mixture after capture has taken place. Thus, it can be seen that as the capture step is facilitated by the expression library members rather than the ligands, the isolation of the solid phase from the rest of the mixture will allow the removal of ligands which have not bound to library members.

The interaction between a capture molecule on the solid phase and the partner molecule or tag on the expression library member is conveniently based on an affinity interaction, although any other appropriate reaction may be used. For example, the members of the expression library can be engineered to express one components of the affinity interaction and the other component can be attached to the solid phase by any appropriate means. Preferred examples of such affinity interactions are antibody-antigen interactions, streptavidin-biotin interactions.

Streptavidin coated beads are commercially available and these can be used to capture ligand-library member conjugates via the presence of a biotin molecule in the library member part of the conjugate. Alternatively, if the expression library is a phage library then capture can readily be achieved using a solid phase to which an antibody to a phage surface protein has been attached, e.g. anti gp8. Alternatively the library members may be engineered to express a tag which is recognized by an affinity partner that facilitates binding to the solid phase. Examples of appropriate tags are c-myc (or other antigen peptide tags) or His tags which can be recognized by anti c-myc antibodies (or other antibodies as appropriate) and metal ions (for example Ni²⁺), respectively, which can in turn facilitate capture to the solid phase.

Where antibodies are used to facilitate capture, these can be attached to the solid phase either directly or indirectly, e.g. via an appropriate secondary or tertiary antibody such as an anti IgG antibody. Capture can be effected in any appropriate way depending on the reagents used. Thus, the antibodies may be attached to the solid phase, which is then brought into contact with the mixture containing ligand-library member complexes. Alternatively, the antibodies may be allowed to bind to the library members in the reaction mixture before the antibodies are bound to the solid phase (e.g. via a secondary antibody) to facilitate ligand-library member capture. Indeed, this is a preferred embodiment of the methods of the present invention. Appropriate capture conditions in terms of temperature and time can readily be determined by a person skilled in the art. However, exemplary conditions may comprise incubation for 10 minutes to 2 hours at room temperature or 4°C (depending on the stability of the ligand or library member), preferably with-gentle rocking, mixing or rotation. The amount of solid phase to include, or the ratio of solid phase to ligand to include, in order to facilitate capture of the ligand - library member complexes can be readily determined by a person skilled in the art.

One or more washing steps can be carried out at any appropriate stage in the screening method. For example, as described above, a washing step (or at least a separation step) is carried out after step (b) in order to remove ligands which have not bound to expression library members. One or more steps of washing the moieties with which the ligands are associated (e.g. washing the cells or the solid phases with which the ligands are associated, might also be carried out after step (a), in order to remove expression library members which have not become bound to ligand. Indeed, such washing steps are preferred. Also, one or more washing steps may be carried out on the moieties with which the ligands are associated, or on the solid phases, at other appropriate times during the course of the method, e.g. to remove non bound entities. How many wash steps to include can readily be determined by a person skilled in the art.

The step of detecting the presence of a ligand is carried out by detecting the ligand directly or at least by detecting an entity (not the library member) with which the ligand is associated. This step provides a contrast to prior art methods where detection generally occurs by detection of the library member which is bound to the ligand rather than the detection of ligand.

Detection of the presence of a ligand (and therefore the presence of a ligand-library member complex, as the non-complexed ligands should have been removed by the capture step as they will not have become bound to the solid phase) is conveniently carried out by detecting the presence of a reporter moiety on or associated with the ligand. Appropriate reporter moieties are discussed above. As described above, said reporter moieties are usually present on or associated with all ligands, i.e. are general labels for all ligands. Thus, as the same reporter moiety is present on (or associated with) each ligand, the presence of a positive signal indicates the presence of a ligand-library member conjugate and therefore a library member which is a candidate binding partner for a ligand. It should-be noted however that if the reporter moiety is present within a cell, i.e. in embodiments where the ligand is a cell surface molecule, then this will have to be exposed, e.g. by cell lysis or other means of cell disruption, before detection can take place.

Appropriate methods of lysing/disrupting cells to expose the reporter moieties which are for example present in the nucleic acids contained within the cells, are well known and described in the art. A preferred lysis buffer for use in the embodiments of the present invention involving eukaryotic cells contains proteinase K, which digests protein and can remove histones from the DNA before the detection step. Other proteases might also be included in the lysis buffers for use in the methods of the present invention.

As described above PCR is the preferred method of detection. However other appropriate methods of reporter moiety detection could be used, e.g. probe hybridisation, fluorescent beads, different size beads, etc (see above). PCR is preferred because of the high sensitivity of the method (even one ligand (or cell) may be detected using PCR), and also because it allows information to be obtained about the ligand, for example sequence information (if for example the ligand is associated with the nucleic acid encoding it), or identification of the ligand (if for example the ligands are tagged with DNA molecules to enable identification of the ligand, e.g. tagged with DNA molecules of different sizes, or tagged with DNA molecules with specific sequence tags which can be used to specifically identify the ligand).

Conveniently the PCR reaction is carried out in solution (although it could also be carried out on a solid phase) and the presence or absence of a PCR product detected in standard ways, such as on an agarose gel (for example the E-gel 96 system, Invitrogen, makes it possible to analyse 96 samples in 20-30 minutes). Generally the samples are well mixed before carrying out the PCR (or other detection step).

Appropriate PCR primers to amplify all or a portion of the reporter moiety are selected according to the sequence of the reporter moiety by methods well known and described in the art. If the nucleic acid content of the assay wells or other assay compartment is appropriate, for example if the ligand is encoded for by nucleic acid which has been transfected into cells using an appropriate expression vector (which contains an appropriate reporter moiety), or the ligand is associated with the DNA encoding it, then PCR primers may b e selected to amplify the nucleic acid encoding the ligand sequence as well as the reporter moiety. This can be advantageous in that the PCR products can be cloned directly into an appropriate vector for sequencing, thereby resulting in the identification of the ligand sequence.

The detection of a positive signal, by PCR or otherwise, is indicative of solid phases (capture solid phases) which require further investigation. For example, if PCR is carried out in solution in the wells of an assay plate, a positive signal is indicative that the well or wells in question contain at least one potential binding partner for a ligand which can then be subjected to further analysis and investigation. For example, the bacterial colonies (or other library members) present in that well can be subjected to thorough screening. e.g. by cell-ELISA to identify the colony (library member) which expressed the candidate binding partner for the ligand.

A positive signal, generated by PCR or other wise, is conveniently determined by comparison to a background signal or a known negative signal. Means and methods of measuring and comparing said positive, background, and/or negative signals are well known to a skilled person

Thus, it can be seen that in a preferred embodiment of the invention, the ligands bound to expression library members are captured onto a particulate and magnetic solid phase and the detection step is carried out by PCR. In a particularly preferred embodiment the ligands are cell surface molecules associated with cell membranes, particularly live tell membranes.

Comparative experiments (see Example 3) have shown that the preferred methods, where cells (in particular cancer cells) are coated with scFv, attached to magnetic beads and the ligand is detected by PCR, are significantly more sensitive than standard methods of cell-ELISA where the detection step is based on the detection of the expression library member not the ligand. The method of the present invention as defined herein (sometimes referred to as the AffiSelect method) required fewer cells to achieve a good signal above background and the signals themselves were stronger and better than those obtained by cell-ELISA.

The methods of the present invention may in volve further additional steps. For example, as discussed briefly above, the expression library may be pre-panned to remove some non-desired expression library members or reduce the complexity of the library. Said pre-panning may be a negative panning step wherein the expression library is contacted with one or more non-relevant entities before step (a) of the method. For example, the expression library may be pre-panned with cells, or membrane fractions thereof, which do not express the desired ligands or express the ligands at a low level. For example, if the method is designed to identify expression library members which bind to a particular type of cancer cell, pre-panning may be carried out by contacting the expression library members with one or more different or irrelevant cell types, e.g. a different type of tumour cell or a non -tumour cell type such as lymphocytes or endothelial cells or other cells which are not of interest. The aim of said negative pre-panning steps is to remove a proportion of the expression library members which will not bind the ligands of interest.

Alternatively, said pre-panning steps can be positive panning steps wherein the expression library is panned/contacted with the target ligand in order to enrich the expression library for members which bind to the target ligands and thereby reduce the complexity of the library to be screened in accordance with the methods of the present invention. One or more pre-panning steps -can be carried out on the same or different relevant or non-relevant entities.

In embodiments of the invention where positive panning is used to enrich the expression library, an appropriate number of rounds, preferably 1, 2, 3 or 4 rounds, of panning with the target ligand are carried out in order to enrich the expression library and to reduce the complexity of the library to be screened. Any appropriate method of panning may be used, e.g. involving attaching the target ligand to a solid phase and panning the library over it. However, a preferred method of panning (in particular where the expression library is a phage display library) will involve the steps of contacting the expression library with the target ligand (e.g. on cells) under conditions such that binding of expression library members to target ligands can occur and then using a modification of the BRASIL method of organic phase separation described in Giordano et al. (2001, Nature Med., 11: 1249-1253) to separate free phages from target ligand bound (e.g. cell bound) phages (such a modified method is described in Example 5 herein). Such a modified BRASIL method includes subjecting the target ligand (e.g. on cells) to at least one, preferably one, two or three, washing steps before separation of the target ligands which have become bound to expression library members from unbound members of the expression library (e.g. unbound phage) by separation through an organic phase, thereby separating candidate binding partners for said target, ligands from other library members. As described above, one or more rounds of panning (contacting and separation), e.g. 1, 2, 3 or 4 rounds of panning may be carried out before the enriched library is screened using the methods of the present invention.

Once the presence of one or more members of the expression library which are candidate binding partners for a ligand have been detected in accordance with the methods of the invention, these can be subjected to further analysis or uses. Said further analysis may involve a further analysis of either or broth the members of the expression library which have bound to the ligands (i.e. the candidate binding partners) or the ligands themselves. Thus, the methods of the invention allow for the screening and identification of both novel expression library members (novel binding partners) and novel ligands, e.g. novel cell surface molecules or proteins such as novel antigens, at both the polypeptide and the nucleic acid level.

Such further analysis generally takes the form of an analysis of single clones which are detected in step (c) of the method. A positive signal in step (c) of the method indicates for example that the assay well or assay compartment in which detection has taken place contains one or more candidate binding partners for the ligand. These candidate binding partners are subjected to further analysis, conveniently by returning to a master plate from which said candidate binding partner was originally picked and analysing the clones (library members) at that position in the master plate by standard methods. Thus, the candidate binding partners are conveniently expressed or produced in isolation from the ligands.

For example for phage expression libraries or other expression libraries wherein the encoded polypeptides are relatively large, such analysis includes, if necessary, cloning the DNA encoding the candidate binding partners into a suitable expression system (if necessary after PCR to convert RNA library members into DNA) which will enable the encoded polypeptides to be expressed in a soluble form-If for example the expression libraries subjected to the screening method were already contained in such an expression system, e.g. where bacterial expression libraries are used, then clearly this step is not necessary.

Once the DNA encoding the candidate binding partners are cloned in a suitable expression vector, the DNA encoding the candidate binding partner can be sequenced or the candidate protein can be expressed in a soluble form and subjected to appropriate binding studies (using the ligand as a target) to further characterize the candidates at the protein level. Appropriate binding studies will depend on the nature of the candidate binding partners and ligand, and include, but are not limited to ELISA, filter screening assays, FACS or immunofluorescence assays, BiaCore affinity measurements or other methods to quantify binding constants, staining tissue slides or cells and other immunohistochemistry methods. Such methods are well established in the literature and one or more of them may be used to analyse the candidate binding partners.

For a peptide library, the associated tags on the candidate binding peptides would be used to identify the sequence of the selected peptides and these could either be synthesised *in vitro* and analysed as described above or the DNA sequence encoding the peptide could be determined and inserted into a suitable expression vector and the expressed peptide analysed as described above.

As a negative control, the candidate binding partners are conveniently also screened against non-ligands, e.g. irrelevant cell types or cell types that do not express the ligand on the surface or express the ligand only at low levels, or irrelevant naked or purified molecules, as appropriate.

In all these methods detection of bound candidate binding partners is facilitated by using reagents which recognize some kind of tag or label on the expression library member. Appropriate tagging and detection systems are well known and described in the art.

In the embodiments of the invention where the ligand is an unknown molecule, e.g. an unknown cell surface molecule, in particular an unknown tumour surface molecule, then the nature of this can also readily be analyzed and the ligand identified using a specific binding partner identified from the expression library as a tool. This is particularly convenient when the binding partner is an antibody molecule, in particular an scFv molecule. For example, as discussed above, such binding partners from the expression library are generally engineered to contain a tag or label which can facilitate detection and can be used for example to analyze the tissue distribution of the unknown molecule, e.g. by binding to tissue sections (Pereira et al., J. Immunol. Methods, 1997, 203:11 -24). Alternatively, the binding partner can be immobilized on a solid phase and used to purify the unknown molecule, e.g. by affinity chromatography. Once purified then the identity of the unknown molecule could be ascertained by appropriate tests dependant on the nature of the molecule. For example, if the unknown molecule is a protein, the purified protein could then be identified by peptide sequencing or mass spectrometry and its encoding DNA sequence determined by cloning and DNA sequencing using methods well known and described in the art. Alternatively, DNA encoding the unknown protein might readily be determined by screening a cDNA library with the identified binding partner. A highly diverse cDNA library from an appropriate source could be used in this regard. However, preferably, a smaller, restricted cDNA library would be used. For example, if the unknown protein was expressed on the surface of a particular cell type, e.g. tumour cells, then a cDNA library (e.g. a cDNA display library such as a phage display library) could conveniently be made from these cells and screened with the binding partner (see for example Ridgway et al., *supra*)*,* for example by panning the display library on solid supports to which the identified binding partner is attached. In addition, if the binding partner identified from the expression library is an antibody then other antibody based assays such as immunoprecipitation, Western blot, expression profiling, immunohistochemistry, etc., could be tarried out in order to isolate or characterize the ligand.

Thus, it can be seen that not only can the methods of the invention be used to select and identify binding partners to ligands from appropriate expression libraries but they can also be used to identify novel and unknown ligands. This is important as it may lead to the identification of novel cellular targets for therapy or diagnosis, as well as novel agents with potential for use in therapy or diagnosis, i.e. the binding partners.

Thus, the present invention also provides a method for isolating and/or identifying an unknown ligand comprising steps-(a) to (c) (and optionally additional steps) as defined herein, (d) isolating one or more expression library members which bind to said unknown ligand and (e) using said library member to isolate and/or identify the ligand to which it binds.

In preferred embodiments of the invention, the AffiSelect method of the present invention, i.e. a method as defined herein, can be used to isolate and identify the unknown ligand. In such embodiments the ligands used in step (a) of the method are a library of ligands (preferably a cDNA library, more preferably derived from the target cell on which the unknown ligand is expressed) expressed in eukaryotic cells (e.g. COS cells) or other suitable cells, and the expression library is one or more binding partners for the unknown ligand, preferably one or more antibody molecules.

Thus, in a yet further aspect, the present invention provides a method for isolating and/or identifying an unknown ligand comprising:
(a) contacting one or more specific binding partners for the unknown ligand in solution with a library (preferably a cDNA library, more preferably a library derived from the target cell on which the unknown ligand is expressed) encoding potential ligands expressed in cells (preferably eukaryotic cells) wherein said binding partners are not attached to a solid phase;
(b) capturing ligands which have become bound to one or more of the specific binding partners onto a solid phase, wherein said capture onto a solid phase is facilitated by the binding partners and wherein said capture onto a solid phase involves the interaction of a capture molecule on the solid phase with a partner molecule or tag associated with binding partners; and
(c) detecting the presence of a ligand, thereby detecting the presence of one or more ligands which are candidate unknown ligands.

The contacting, capture and detection steps are carried out as described elsewhere herein. A particularly preferred method of detection is PCR, preferably using primers from the ligand library vector sequence (rather than for example a household gene) in order to allow the immediate amplification of the sequence of the ligand that is recognised by the specific binding partner. The PCR products can then be cloned directly into an appropriate vector for DNA sequencing and further characterisation of the encoded protein.

Methods of the invention can thus be used to select, identify or isolate binding partners for a ligand, or a novel ligand per se, which can then be isolated, produced or manufactured for various downstream uses. As such, binding proteins or ligands identified or selected using the methods of the invention are disclosed herein. Thus, a further aspect of the present invention provides a method of selecting, identifying and/or isolating a library member which is a specific binding partner for a ligand or a ligand per se from an expression library, said method comprising the steps of screening an expression library using steps (a) to (c) as defined above to select molecules which display certain properties and (e) identifying and/or isolating the relevant library member (s) which are specific binding partners for the ligand and optionally (f) using said library members to identify the ligand to which it binds.

Once appropriate nucleic acid fragments encoding binding partners or ligands with particular properties have been identified, the nucleic acids encoding the polypeptides can, if desired, be subjected to affinity maturation, for example to try and identify binding partners with further improved properties. Such affinity maturation can be performed by carrying out any conventional form of mutagenesis, including but not limited to the addition, deletion and/or substitution of one or more nucleotides in a controlled (e.g. site directed mutagenesis) or random manner, error-prone PCR, domain swapping, cassette mutagenesis and chain shuffling, etc., prior to rescreening.

When one or more binding partners or ligands have been selected, identified, isolated and/or purified using the methods of the invention, these candidates, or a component, fragment, variant, or derivative thereof may be manufactured and if desired formulated with at least one pharmaceutically acceptable carrier or excipient. Such manufactured molecules, or components, fragments, variants, or derivatives thereof, are also disclosed herein. Alternatively, these molecules may take the form of nucleic acids encoding said protein molecules, which nucleic acids may in turn be incorporated into an appropriate expression vector and/or be contained in a suitable host cell. Thus, nucleic acid molecules encoding said binding partners or ligands, or expression vectors containing said nucleic acid molecules are disclosed herein.

Once a particular binding partner or ligand, or a component, fragment, variant, or derivative thereof, has been selected, identified, etc., in accordance with the present invention, the expression vector encoding the selected binding partner or ligand can readily be used (or adapted for use) to produce sufficient quantities of the molecule by expression in appropriate host cells or systems and isolating the binding molecules from the host cell or system or from the growth medium or supernatant thereof, as appropriate. Alternatively, said binding molecules or ligands may be produced by other appropriate methods, e.g. by chemical synthesis of the nucleic acid encoding the binding molecule and expression in a suitable host or in an *in vitro* transcription system.

Disclosed herein is a method of manufacturing a specific binding partner or a ligand comprising the steps of identifying or selecting a specific binding partner for a ligand or the ligand per se according to the methods of the invention as described above, manufacturing said identified binding partner or ligand, or a component, fragment, variant, or derivative thereof and optionally formulating said manufactured binding partner or ligand with at least one pharmaceutically acceptable carrier or excipient.

Variants or derivatives of a binding partner or ligand include peptoid equivalents, molecules with a non-peptidic synthetic backbone and polypeptides related to or derived from the original identified polypeptide wherein the amino acid sequence has been modified by single or multiple amino acid substitutions, additions and/or deletions which may alternatively or additionally include the substitution with or addition of amino acids which have been chemically modified, e.g. by deglycosylation or glycosylation. Conveniently, such derivatives or variants may have at least 60, 70, 80, 90, 95 or 99% sequence identity to the original polypeptide from which they are derived.

Where the binding partner is an antibody molecule, variants or derivatives further include the conversion of one format of antibody molecule into another format (e.g. conversion from Fab to scFv or vice versa, or the conversion between any format of antibody molecules described elsewhere herein), or the conversion of an antibody molecule to a particular class of antibody molecule (e.g. the conversion of an antibody molecule to IgG or a subclass thereof, e.g. IgG1 or IgG3, which are particularly suitable for therapeutic antibodies).

Variants or derivatives further include the association of binding partner molecules or ligands with further functional components which may for example be useful in the downstream applications of said binding partners or ligands. For example the binding partners or ligands may be associated with components which target them to a particular site in the body, or detectable moieties useful for example in imaging or other diagnostic applications.

Clearly, the main requirement for such components, fragments, variants, or derivative binding partner molecules or ligands is that they retain their original functional activity in terms of binding ability or have improved functional activity.

The binding partner molecules (preferably antibody molecules) or ligands isolated, detected, selected, identified or manufactured using the methods of the present invention may be used in any methods where binding partners specific to a ligand (for example antibodies specific to a particular antigen) are required. Thus, the binding partners (preferably antibody molecules) or ligands can be used as molecular tools and a further aspect of the invention provides a reagent which comprises such binding partner molecules or ligand molecules as defined herein. In addition, such molecules can be used for *in vivo* therapeutic or prophylactic applications, *in vivo* or *in vitro* diagnostic applications, or *in vitro* assays.

The binding partners may be used in therapeutic applications, either in the form isolated from the expression libraries or engineered or converted forms, e.g. it may be desirable to convert an scFv molecule to an IgG or to multimerize peptides. The therapeutic effect might be effected by inducing a biological activity upon the therapeutic molecule showing agonistic or antagonistic binding to the ligand, e.g. inducing apoptosis (for example of cancer or viral infected cells), inhibiting growth or stimulating detachment from matrix by blocking the natural ligand from binding (to thereby inhibit growth and/or spread of tumours). Such therapeutic effects might be achieved due to properties of the ligand itself, or a multimer thereof(some receptors are activated by cross linking them).

Where the binding partners selected or identified, etc., are antibody polypeptides then these can be used for *in vivo* therapeutic and prophylactic applications, e.g. to confer passive immunity to particularly susceptible individuals (e.g. immunocompromised patients, small children, the fetus of pregnant women, people in endemic areas for disease, etc). For example if the antibodies are capable of neutralizing infective or disease related agents then these can be administered to an appropriate subject to combat disease. Alternatively, antibodies (or other forms of binding partner) can be attached to other therapeutically effective molecules, e.g. to cytotoxic agents (small molecule or protein), pre-toxin or other drugs and targeted to disease tissue or specific cell types, e.g. tumour cells or virus infected cells. Further therapeutic effects might be achieved by other functions engineered into the agent to be administered like the ability to activate macrophages, -complement or cytotoxic T cells conferred for example after changing an scFv to an Ig, generation of bispecific molecules, e.g. linking tumour cells and killer cells.

Alternatively such antibodies (or indeed other types of polypeptide which interact with ligands associated with particular tissue or body sites, or cell types, e.g. tumour cells or virus infected cells) can be conjugated to labels, e.g. dyes, fluorescent or radioactive labels or enzymatically detectable labels, and used for *in vitro* or *in vivo* diagnosis, for example by imaging or standard immunohistochemical procedures. Other preferred uses include theranostic uses (i.e. antibodies or binding partners used in both diagnosis and therapy). In addition, such antibody molecules or other binding partners may be used in affinity chromatography procedures to isolate ligands.

In particular, antibodies to cell surface expressed proteins provide a well described starting point for the successful development of new diagnostic and therapeutic drugs. This is particularly the case in the cancer field, where the knowledge of specific cell surface markers as well as antibodies binding to them, is a clear bottleneck in drug development, and the methods of the invention can be used to identify or select both novel cell surface markers (ligands) and antibodies (binding partners). Currently, only a very limited number of cell surface expressed tumor-associated or tumor-specific epitopes are known. Thus, every new epitope of such type- and of course specific antibodies binding to them - would open new possibilities in the treatment of cancer. Possible applications span from naked whole IgG antibody products for ADCC-based therapy, over recombinant oligo-specific/oligo-valent constructs, to fusion-protein immunotoxins and radio-labeled antibody fragments for tissue-specific and targeted therapy, as well as dye- or radio-coupled antibodies for *in vitro* and *in vivo* diagnosis.

The ligands, or fragments thereof could be used as vaccines, in particular vaccines for use in the treatment for cancers and infectious diseases (depending of course on the ligand in question). The ligand may be used as target for agonists and antagonists, in form of peptides, proteins or small molecule drugs, which may for example block or induce functions like apoptosis or regulating cell growth. The ligands may also be used as a target for further screening, to identify even more molecules able to do some of the things described above. In some cases, the ligands or fragments thereof might also have an effect in itself, like competitively binding of molecules naturally binding to it.

Suitable and appropriate adaptations of the antibody molecules, if necessary for such uses, e.g. the conversion to IgG1 or IgG3 classes for therapy, the incorporation or addition of an appropriate label for imaging, etc., would be well known to a person skilled in the art.

The most suitable antibodies for the various uses described above can be readily identified using appropriate tests which can be designed by a person skilled in the art. For example, in applications where high affinity or avidity of an antibody or a binding partner is important these criteria can readily be tested in candidate antibodies using standard assay techniques (e.g. Biacore assays). In addition, where antibodies against a particular infectious agent, e.g. a bacteria, virus etc., have been identified, appropriate tests can be carried out to assess which antibodies are most effective to neutralise the infectious agent. For example, in the case of bacteria, bactericidal and opsonophagocytic activity against the bacteria in question can be assessed. Similarly, in the case of viruses, viral neutralisation studies can be carried out to identify the best candidates.

A yet further aspect of the invention thus provides the use of an antibody expression library as described herein to isolate, detect, identify, select or manufacture one or more antibody molecules which bind specifically to one or more target antigens, for example one or more target antigens which are associated with particular diseases, cells, tissues or foreign agents. This could for example be carried out by screening the antibody expression libraries with the target antigens.

Disclosed herein are such isolated, detected, identified, selected or manufactured antibody molecules (or other binding partners) for use in therapy or *in vivo* diagnosis or for use in any of the other applications mentioned above. Also disclosed is the use of such antibody molecules

(or other binding partners), or ligands, in the manufacture of a medicament or composition for use in therapy (in particular therapy for cancer or infectious diseases) or *in vivo* diagnosis or for use in any of the other applications mentioned above. Methods of treatment of a patient comprising the administration of an appropriate dose of such an antibody molecule (or other binding partner) are also disclosed.

When said antibody molecules (or other binding partners), or ligands, are used in the above described uses and methods then these may be administered in any appropriate way. For example such antibody molecules (or other binding partners) may be administered locally at the site where action is required or may be attached or otherwise associated with entities which will facilitate the targeting of the antibody molecules (or other binding partners), or ligands, to an appropriate location in the body.

Pharmaceutical compositions comprising the antibody molecules (or other binding partners), or ligands, as defined herein, together with one or more pharmaceutically acceptable carriers or excipients are also disclosed.

Also disclosed are methods of diagnosis or imaging of a patient comprising the administration of an appropriate amount of an antibody molecule (or other binding partner), or ligands, as defined herein to the patient and detecting the presence, location and/or amount of the antibody molecule in the patient.

The antibody molecules (or other binding partners), or ligands, identified, selected, etc., from the expression libraries of the invention may equally be used in methods of diagnosis which are carried out *in vitro,* if appropriate, e.g. carried out on a tissue sample or some other kind of sample, e.g. blood, obtained or derived from a patient.

Preferred diseases to be treated or diagnosed, etc., are cancer and infectious diseases caused by infectious agents, inflammatory diseases, autoimmune diseases or degenerative diseases.

The terms "therapy" or "treatment" as used herein include prophylactic therapy. The terms "therapy" and "treatment" include combating or cure of disease or infections but also include the controlling or alleviation of disease or infection or the symptoms associated therewith.

The methods of the invention can be used for large scale screening, i.e. can be used to simultaneously screen expression libraries with up to 500 000 different members, or libraries with 1x10⁶ or more members. Conveniently for such large scale screening the expression library can be provided in the form of bacterial colonies on one or more solid supports such as a filter or plate, and/or colonies grown in individual wells of one or more assay plates (e.g. one or more 384 well or 96 well assay plates). For example, 30 000 to 300 000 colonies can readily be grown on filters using methods well known to a person skilled in the art, and these colonies (or a subset thereof) can then be picked and transferred to an assay plate where the colonies can be further grown in suspension and then induced or otherwise treated to express the library members at the polypeptide level ready for screening in accordance with the methods of the present invention. If the library members are expressed internally, i.e. the expression library is not a display library, then lysis of the cells, e.g. the bacterial cells, is also required in order that the expressed library members can be available and accessible for screening.

One colony (or one expression library member) can be picked into or can be present in one well of an assay plate (or other appropriate assay compartment), and indeed it is generally desired that assay plates with only single clones (single library members) in each well are obtained initially in order to simplify the downstream rescreening processes. However, to facilitate large scale screening multiple colonies (multiple expression library members) can be picked into or can be present in the same well of an assay plate (or other appropriate assay compartment) and multiple library members can be expressed in the same well of an assay plate. For example, bacteria from up to 100, 300, 500, or 1000 colonies can be pooled into one well of an assay plate and induced to express the library members. After lysis, if necessary, the expression libraries are then ready to be brought into contact with the ligands in accordance with the present invention. Alternatively, and preferably, expression of the library members and lysis (if necessary) can be carried out whilst the colonies are in individual wells and before the library members are pooled in the same well. This is preferred because the amplification and expression of multiple colonies in the same well might lead to potential problems, for example if dominant or toxic library members are present.

When multiple colonies are present in the same well, conveniently it is desirable that colonies from a whole assay plate or from a defined region of a filter are placed into a single well. For example, individual bacterial colonies may be picked, grown and induced (and optionally lysed) in 384 well plates (e.g. 90-100 plates makes 35,000-38,000 colonies). From each well, 1-3 µl of bacterial medium (which can then be lysed) or periplasmic lysate with expressed polypeptides are pooled with the same volume from the other wells into one single deep-well of a 96 deep-well plate (making a pooled volume of 768 µl). This can be repeated with another 384 well plate giving finally 384 x 96 clones (36,864) to be analysed in one single 96 deep-well plate. Indeed the examples show how it is possible to detect single positive wells among 384 pooled wells. In addition, it can be seen how this method can readily be expanded to screen ∼370 000 (384 x 96 x 10) different colonies in a relatively short time period (1 to 2 weeks).

It should be noted that in the embodiments of the invention which do not involve the use of bacterial colonies for expression, e.g. when the expression libraries are *in vitro* display libraries such as ribosome or covalent or non-covalent display libraries the same principles apply, i.e. an individual library member is ideally present in a single well of an assay plate which can be induced and screened as individual clones or can be induced and pooled for screening or can be pooled and then induced for screening.

Appropriate induction/expression and lysis methods to obtain soluble/insoluble protein for screening are determined by the nature of the particular expression library selected for use in the present invention and are well known and described in the art. For example, the Relay™ 96 Protein Screening System (Invitrogen) is particularly appropriate for use in a 96 well or 384 well format as it allows the extraction of soluble proteins (and, if desired, insoluble proteins) from the same sample in 30 minutes.

Once the expression library is in an appropriate format for assay, e.g. has been divided into multiple wells of an assay plate and the polypeptides have been expressed in such a form that they are accessible for binding, the expression library is brought into contact with one or more ligands as described elsewhere herein under appropriate conditions to facilitate binding between the polypeptides of the expression library and the ligands.

After contacting the expression library in the wells with the appropriate ligands under appropriate condition and capturing of the ligand-expression library complexes on a solid phase within the wells as described elsewhere herein, e.g. using magnetic beads, PCR of the reporter moiety can be carried out in the wells to assess for potential positives. A positive signal for one 96 well implies that there must be at least one positive among the 384 clone. These clones (corresponding to one 384 well plate) must be reanalysed (subscreened). However, this has reduced the number of potential clones from 36,864 to 384.

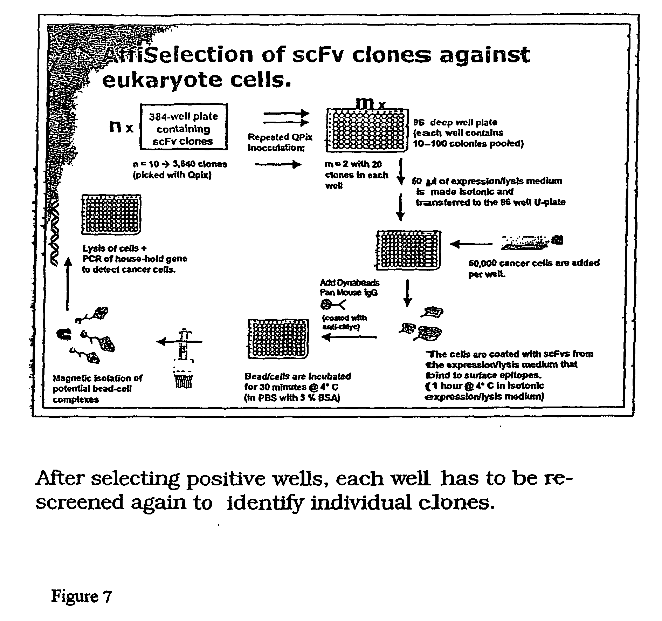

A smaller scale version of this method, where 10-100 colonies are pooled in each well of a 96 well plate is shown as a schematic in Figure 7, thereby allowing the screening of 1000 - 10 000 colonies per 96 well plate. In this specific example (although it will be appreciated that this method can be generalized), the scFv induction/lysis medium is made isotonic and transferred to a 96 well plate with 50 000 cancer cells per well. The cells are coated with scFvs for 1 hour at 4°C. Antibody coated magnetic beads towards scFv tags are added and bead-cell complexes are isolated. If there are some antibodies binding to cells, the corresponding cancer cells will be isolated and detected by PCR. A positive PCR signal thus identifies a small pool of scFv candidates (10 - 100) for further investigation. As described in more detail later, the scale of the method can be selected as appropriate based on the number of colonies it is desired to screen (i.e. the size of the expression library to be screened) and optionally an estimate of the number of positives likely to be present in the expression library.

A yet further aspect of the present invention provides a method of screening a library of molecules to identify and/or select one or more members thereof which are candidate binding partners for one or more ligands, comprising:
(i) pooling a number of bacterial clones which are capable of expressing expression library members, or pooling a number of expression library members which have been produced by a number of bacterial clones, in a single well of an assay plate (or other appropriate assay compartment);
(ii) if a number of bacterial clones are pooled in step (i), inducing the bacterial clones to express said expression library members;
(iii) contacting said expression library members with one or more ligands wherein said expression library is not attached to a solid phase;
(iv) determining positive assay wells in which one or more expression library members have become bound to ligand wherein said determining step is carried out in accordance with steps (b) and (c) as defined above;
(v) carrying out a selective expansion of the clones which are present in the positive assay wells, and repeating steps (iii) and (iv);
(vi) identifying one or more bacterial clones which express the binding partners for said ligand

Such a screening method (which may also be referred to as cross-screening) is particularly suitable for screening libraries which contain only a low percentage of positive clones. As will be described in more detail below, this pooling (which may also be referred to as "compression") and selective expansion method can advantageously be used to screen up to 2 million bacterial clones and possibly ten times that number.

The expression libraries for use in these methods can be any protein expression library as described elsewhere herein. Preferably the expression libraries are antibody expression libraries, more preferably scFv libraries. In the methods of this aspect, it is preferred that pooling (compression) of library members which have already been expressed is carried out.

The determination (determining) step is carried out in accordance with steps (b) and (c) of the methods of the invention described above, i.e. by capturing ligands which have become bound to one or more members of the expression library onto a solid phase and detecting the presence of a ligand, thereby detecting the presence of one or more members of the expression library which are candidate binding partners for the ligand. Thus, it can be seen that this method represents an exemplary method by which the methods of screening as defined in steps (a) to (c) discussed above (i.e. the AffiSelect method) can be carried out. Steps (b) and (c) as described above are used, so all the preferred embodiments and details described above with regard to carrying out these steps apply equally to the above screening method involving the pooling of bacterial colonies or expression library members.

The selective expansion step (v) generally involves replating the clones which are present in the positive assay wells (compartment) into a less compressed (less pooled) format, for example into an increased number of assay wells (compartments). Preferably said expansion step results in each clone being present in a single assay well (compartment). Such a selective expansion step is conveniently carried out by returning to the original (pre-pooling, pre-compression) source of clones for the positive wells and replating these clones in a less compressed format.

The number, if any, of selective expansion steps (v) (and repeats of steps (iii) and (iv)) to be carried out can readily be determined by a person skilled in the art. For example such steps could be carried out until each clone is present in a single assay well to facilitate an easier identification step (vi).

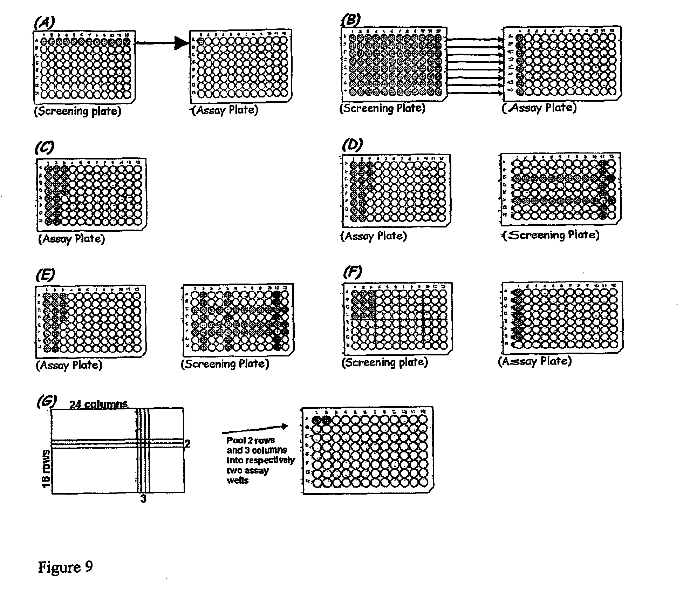

The identification step (vi) can conveniently be carried out by returning to the original (pre-pooling, pre-compression) source of clones for the positive wells (see for example the intersection wells of Figure 9D) and carrying out a single clone analysis using standard methods as described elsewhere herein to characterise the binding partners at the nucleic acid and protein level.

The bacterial clones to be pooled and screened (or which produce the expression library members to be pooled and screened) in the screening methods of this aspect of the invention may be derived from any appropriate source, but conveniently are initially present as part of a confluent layer of bacteria, or individual colonies of bacteria on plates or filters. Alternatively, if the number of colonies to be screened is relatively small, then these colonies can be present in individual wells of appropriate assay plates. If the clones are growing as a confluent layer or as individual colonies on a plate or filter then conveniently clones for testing in the screening method can be picked using appropriate robotic technology (e.g. Qpix) or by hand.

As a specific example, if a confluent layer of bacteria is used, each pin of the robotic picking head is likely to pick up 5 - 50 colonies which can then be transferred to single wells of an appropriate assay plate, e.g. a 96 well or 384 well assay plate. By transferring clones and amplifying them in single wells of a 384-well plate it is possible to simultaneously screen 3,800-19,000 clones per 384-palate. Using e.g. one-hundred to two-hundred 384-well plates it is possible to screen up to 4 million clones.

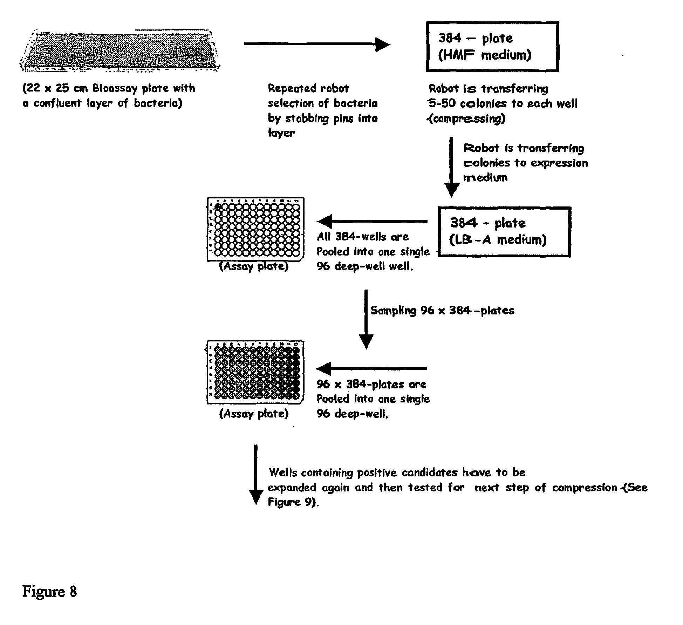

Using these 384 well plates as a source of bacterial colonies, step (i) of the method can be-carried out by pooling-(compressing) these clones into 1-2 single 96 deep-well plates by pooling colonies from each well of a single 384 well plate into one single well of a 96 well plate (Figure 8). Steps (ii) to (vi) of the method can then be carried out. A positive well determined in step (iv) of the method corresponds to ∼ 19,000 clones or one specific 384 well plate. This specific 384 well plate might then be cross screened as described in Figure 9G (by selective compression of rows and columns (which is a selective expansion step in this particular example), and sub-screening by carrying out steps (iii) and (iv) of the claimed method (which also requires rescreening of the 6 wells at the points of intersection). After a single well of the 384 well plate has been identified, still ∼ 50 clones have to be screened. This final piece of screening can be carried out by plating out all the bacteria of the well and performing another sub-screening of individual picked colonies. The amount of time it requires to screen 1x10⁶ clones will be about 1-2 weeks.

In order to determine the most efficient way to carry out the screening method then preferably a pre-screening step is carried out to assess the percentage of positive clones. This can be done by any appropriate method. However, conveniently this can be done by pooling randomly selected clones, e.g. by pooling 10, 100 or up to 100 000 clones, in one assay well, and performing the AffiSelect method (as described herein) to predict the hit ratio.

If the percentage of positives is as low as 0.0001 % (or 1 clone per 100, 000 - 1 x 10⁶ colonies), or even lower, then the method described above of pooling all 384 samples from each well of each plate (as described in Figure 8), is appropriate. For example, 1-2 million clones are sampled from a confluent layer of bacteria, and induced in 384-well plates (each well containing about 50 bacteria, 100 x 384 plates). Each 384-plate is then compressed into one single well of a deep-well plate and assayed against target using magnetic beads and PCR. Positive regions (containing approximately 20 000 clones) are expanded again and sub-screened as described above by reference to Figure 9G, i.e. by pooling 2 rows and 3 columns (requires rescreening of 6 positions at intersection) to eventually identify a single well of 50 clones, which can be plated out and screened individually.

For 0.01 % to 0.1 % positives (or lower) several 384-plates are ideally screened, pooling each 2^{nd} row and 3^{rd} column as described in Figure 9G.

For 0.1-3 % positives it is wise to screen whole 384-plates by pooling individual rows and columns (16+24). This may avoid re-screening. However, if the 384 plate originates from a larger screen of 10⁶ clones, each well of the 384-plate contains more colonies, and has to be replated to identify single clones.

For 0.5 % to 3 % positives a more convenient solution is to pool all rows and columns of the 96 well plate (as described in panel (A)-(C) of Figure 9) to 20 Assay positions.

For 2-10% positives, it might be advisable to screen small regions as in panel (F) of Figure 9. For example, dividing one 96 well screening plate into eight regions each containing 7 rows & columns, compresses the number of candidates to be tested to 56.

For more than 10% positives all clones should be screened individually without any compression.

As indicated above, the method of carrying out the contacting and determination step is the same as described elsewhere herein. For example, eukaryotic cells or antigens (ligands) are coated (1-2 hours) with scFvs expressed and excreted to bacterial medium. The ligand (cells or DNA-labelled antigen) is washed and antibodies against the cMyc-tag expressed on the scFv (secondary antibodies) are added. Using magnetic beads with tag-directed antibodies (cMyc), only if the scFv binds to target, is the complex between scFv and ligand (eukaryotic cells or DNA-labelled antigen) isolated and purified with magnetic beads. If there are no scFvs binding to target, no complexes will be isolated, and PCR will be negative. If, however, there is a single positive scFv (among up to 20,000 other scFvs) which is not removed by washing, PCR of scFv-target complexes will contain DNA with a positive amplification result. PCR of a household gene of the eukaryotic cell is a reporter showing that there must have been some antibodies in the well expressing scFvs binding to antigen. A positive signal for one 96 well implies that there must be at least one positive among the 384 clones. These clones (corresponding to one 384-plate) must be reanalysed (sub-screened).

The invention will now be described in more detail in the following non-limited Examples with reference to the following Figures which show:

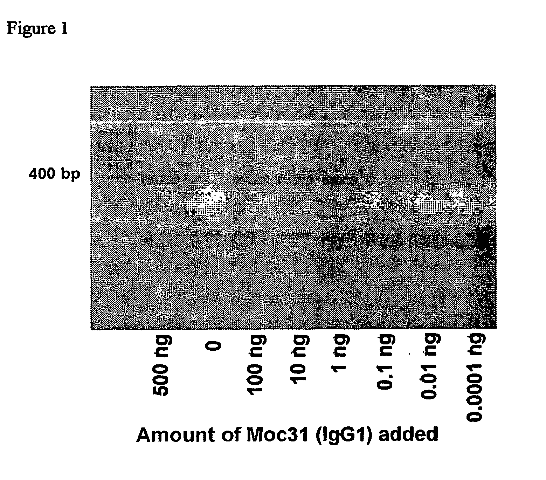

Figure 1: In solution capturing and detection of the mouse IgG Moc31 binding to PM-1 cells. The figure shows a 2 % agarose gel analysis of the β-actin fragment PCR product from PM-1 cells - the final step after coating the cells with Moc31 and magnetic enrichment. The bead:cell ratio was kept as 10:1 using 50, 000 PM-1 cells and a capture volume of 50 µl 3 % BSA in PBS at 4° C for 30 minutes on a shaker.

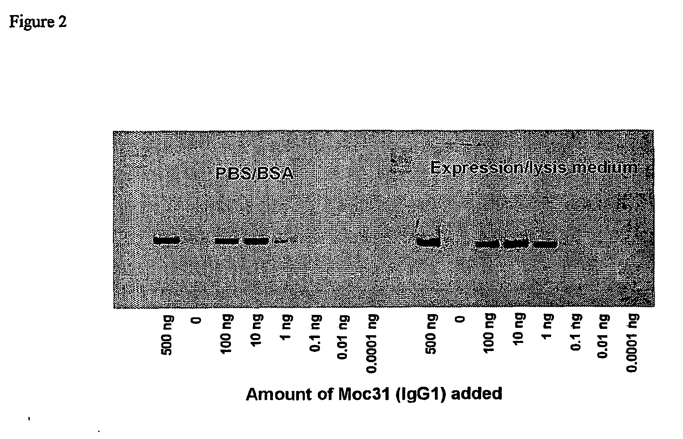

Figure 2: Capturing and detection of the mouse IgG Moc31 binding to PM-1 cells in PBS/3%BSA or in isotonic bacterial expression medium. The figure shows a 2 % agarose gel analysis of the β-action fragment PCR product from PM-1 cells-the final step after coating the cells with Moc31 and magnetic enrichment as described in Figure 1. Moc31 was either added to 3 % BSA/PBS or to expression/lysis medium (LB with 100 µM IPTG, 100 µg/ml ampillicillin and 2 mg/ml Lysozyme made isotonic (∼140 mM NaCl) with sterile water and added 0.3 % B SA).

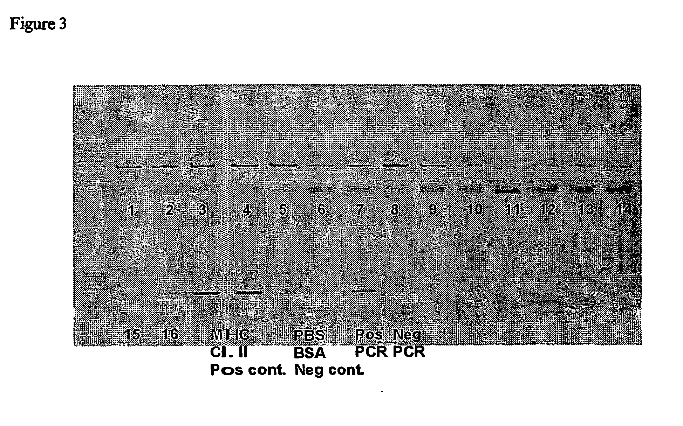

Figure 3: Capturing and detection of M13 phages carrying Different scFvs against surface epitopes of A-549 cells in PBS/3%BSA. The figure shows a gel analysis (2 % agarose) of the β-action fragment PCR product from A-549 cells. This is the final step after coating and washing the A-549 cells (100,000) sequentially with M-13 phages and anti gp8 antibodies (1:1000), and capturing complexes with Pan Mouse IgG immobilised on Dynabeads M-450. As positive control MHC class I antibodies (MHC Cl. I Pos. cont., 1:25) were also added to cells or no antibodies at all (PBS BSA Neg control). In addition positive (β-actin gene) and negative PCR controls (no DNA) were used.

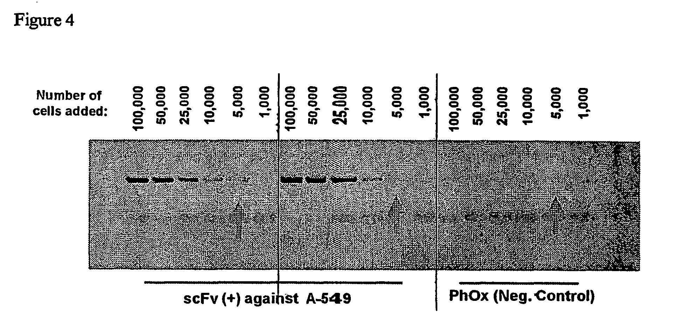

Figure 4: Lowering the number of A-549 cells that can be used for indirect detection of ScFv antibodies against cell surface epitopes. The Figure shows a gel analysis (2 % agarose) of the β-actin fragment PCR product from A-549 cells. This is the final step after coating and washing the A-549 cells (from 1,000 up to 100,000 cells) sequentially with scFv and anti-cMyc antibodies, and capturing coated A-549 cells with Pan Mouse IgG Dynabeads. In this experiment PhOx scFv is a negative control antibody and should not bind to cells.

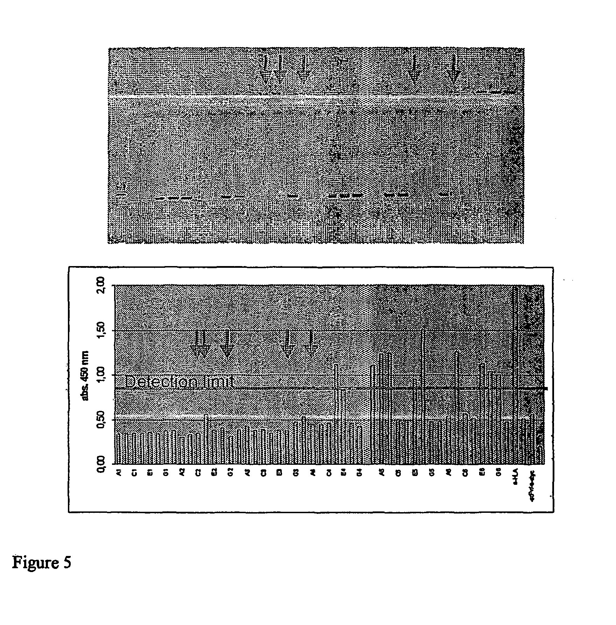

Figure 5: Comparing cell-ELISA with AffiSelect. Top panel of the figure shows a gel analysis (2 % agarose) of the β-actin fragment PCR product from A-549 cells (AffiSelect). This is the final step after coating sequentially with scFv and anti-cMyc antibodies, washing the A-549 cells (100,000 cells), and capturing coated A-549 cells with Pan Mouse IgG Dynabeads. Bottom panel is cell ELISA of the same clones (Methods), but with 200,000 cells.

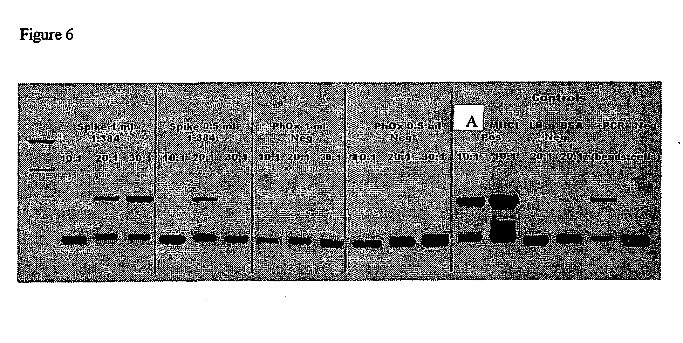

Figure 6: Detecting spiked scFv antibodies binding to A-549 cells with AffiSelect. The figure shows a gel analysis (2 % agarose) of the β-actin fragment PCR product from A-549 cells (AffiSelect). This is the final step after coating and washing the A-549 cells (100,000 cells) sequentially with scFv and anti-cMyc antibodies, and capturing coated A-549 cells with Pan Mouse IgG Dynabeads. Different bead:cell ratios were used during capture, from 10:1 to 30:1 as indicated. Samples labelled "Spike" contain a 1:384 ratio of the positive control scFv A ('A';2 µl) and the negative control PhOx (768 µl) and adjusted with Milli Q water to get isotonic conditions. The capturing volume (volume of beads + cells) is also shown.
Figure 7: A schematic of the method of the invention screening scFv clones against eukaryotic cells.
Figure 8: Cross-screening of a large number of clones into 384-wells. (Library with a low hit-rate of positives: 1/500,000 or 1/1,000,000). This figure shows selective compression and expansion of clones. Sampling 96 x 384 plates into one single 96 deep-well plate makes it possible to sample 1.8 million clones. A positive well of the assay plate corresponds to - 19,000 clones or one specific 384-plate. This plate has to be cross-screened as described in Figure 9G (by selective compression of rows & columns). After the single well of the 384-plate has been identified, still ∼50 clones have to be screened. This last piece of screening will take place by plating out all the bacteria of the well and performing another sub-screening of individual picked colonies. The amount of time it requires to screen 10⁶ clones will be about 1-2 weeks.
Figure 9: Cross-screening of 96 and 384-well plates. (A) Pooling a small volume (1-5 µl) from each well of row A of the screening plate onto one single well (A1) of the assay plate. (B) Pooling similarly small volumes as shown in (A) for all rows A-H. Eight rows of the screening plate can be represented by 8 wells on an assay plate. (C) Pooling the 12 different column entries of the screening plate into 12 separate wells on the assay plate (wells A2-H2 and A3-D3). (D) To identify clones on the screening plate, the assay plate revealed two positive positions on rows 3 and 6 and one for-column 11 (i.e. wells C1, F1 and C3 were positive) identifying two true positives on the screening plate (seen at wells C11 and F11). However, having more positives as described in (E), the screening plate also shows false positives (seen at wells C2, C11, E5, E11, F2 and F5), making it necessary to subscreen all candidates in order to pick the right positive clone. In this case it may be better to screen 8 regions of the assay plate as described in (F). Respective rows (4) and columns (3) for each region may be pooled into 7 wells on the assay plate. Finally screening a 384-well plate by pooling rows and columns is described in (G). Each 384-plate is represented by only 16 wells on the assay plate. This requires however a re-screening of the 6 positions in the middle (the point of intersection).

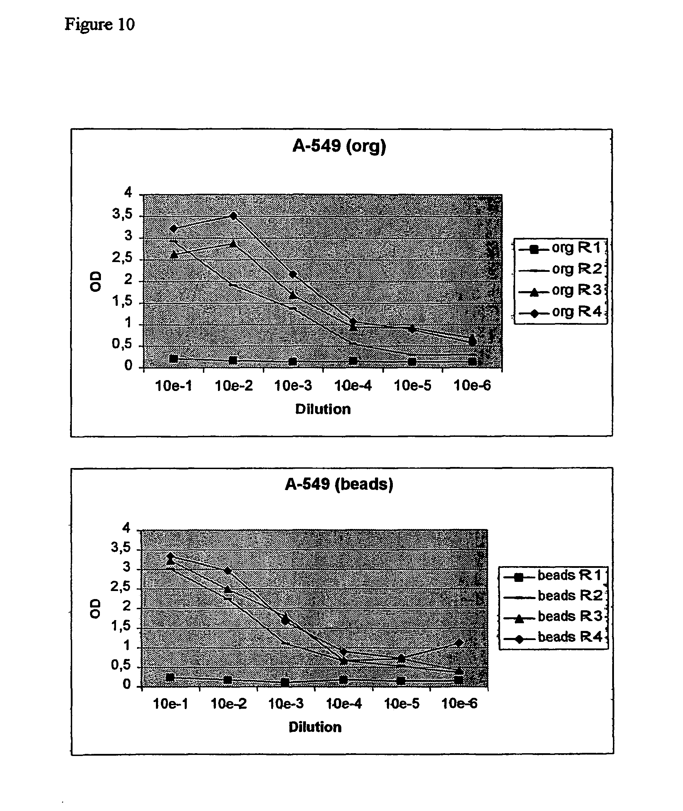

Figure 10 shows polyclonal phage ELISA on lung carcinoma cell line A-549 with phage dilutions after panning round 1-4 (R1-R4) using organic phase centrifugation (org, modified BRASIL) and lectin coated magnetic beads (beads).

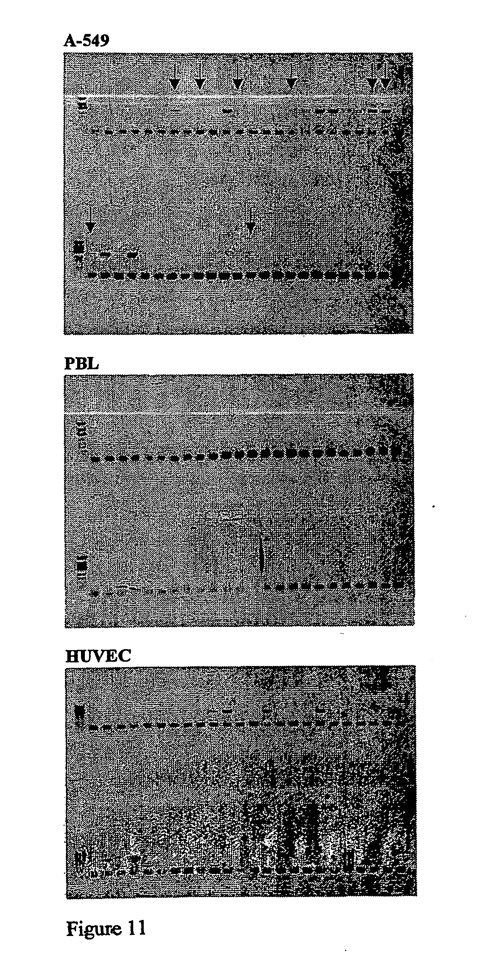

Figure 11 shows an example of AffiSelect PCR results showing amplified beta-actin DNA in cases where the tested scFv expression was binding to cells (A-549, HUVEC and PBL). Results of the same scFv expression samples are shown (in the same order) on the three cell types. Arrows mark the scFv expression samples that showed tumor-specific DNA amplification.

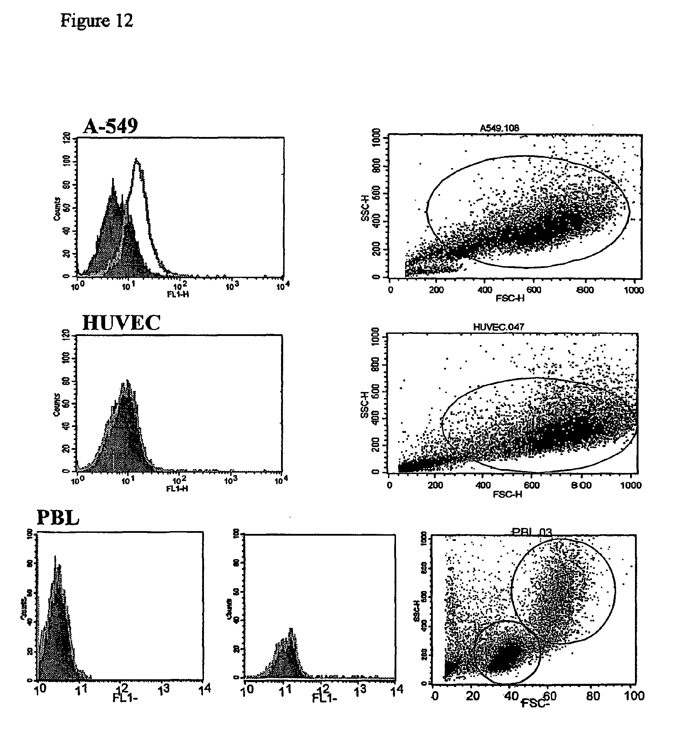

Figure 12 shows a FACS result of one of the scFv found after panning using 2 washes and organic phase centrifugation. The scFv was tested on the same-cell types that were used in the panning and pre-panning steps (A-549, HUVEC and PBL).

### EXAMPLES

### Example 1:

### Capturing and detecting mAb, scFv and phage (library members) binding to surface epitopes of eukaryotic cell ligands with immunomagnetic separation and PCR of a reporter gene of the cell.

### Abstract:

This example is illustrating the selection and detection process. Eukaryotic A-549 (gift from Viventia, Inc., ATCC CCL-185) or PM-1 cells (gift from The Norwegian Radium Hospital) (with cell surface ligands) were coated with either mAbs, scFvs or phages (affinity members). If the affinity members are binding to the cell surface ligands, a linking moiety can be made by the affinity member which makes it easy to capture and detect those ligands having an affinity member associated. This was shown for all affinity members tested.

### Materials and Methods:

The mouse Moc-31 antibody, binding to a 38 kDa transmembrane EGP-2 epithelial cell adhesion molecule (De Leij, L., Helfrich, W., Stein, R., and Mattes, M. J. (1994) SCLC-cluster-2 antibodies detect the pancarcinoma/epithelial glycoprotein EGP-2. Int. J. Cancer SuppL, 8, 60-63.) was a kind gift from The Norwegian Radiumhospital. Mouse antiHuman HLA-ABC (MHC ClassI) was purchased from Dako, and proteinase K, B SA (Fraction V), Tween 20 and NP-40 from Sigma. Proteinase K stock solution was made in 50 mM Tris-HCl pH 8.0 added 1.5 mM CaCl₂ to a concentration of 20 mg/ml and stored in aliquots at -20° C. Sterile Milli Q purified water was applied in all solutions, and all solutions were either sterilised by autoclaving or by 0.22 µm filtration.

The eukaryotic human breast carcinoma cell line PM-1 was counted in Bürker Haemocytometer and adjusted to 3.2.x 10⁶ cell/ml in PBS with 3 % BSA. The cells were washed in 3 % BSA and centrifuged at 400-500 x g for 5 minutes in a Greiner 96-well U-plate, each well contained 50,000-100,000 PM-1 cells. Primary antibodies (binding to cell epitopes) in PBS/3 % BSA were then added to the cells (100 µl volume) and incubated for 60 minutes at 4° C on a Heidolph Unimax 1010 plate shaker at 280 rpm to avoid sedimentation. Then the cells were washed twice in 200 µl PBS /3 % BSA. If the primary antibodies also contain tags (e,g. a scFv antibody with a cMyc and His₆ tags), secondary antibodies were diluted in PBS /3 % BSA and added to the cells and incubated for another 60 minutes as above (e.g. mouse anti-cmyc (Invitrogen, 1:500) or mouse anti-His (Invitrogen, 1:500)). If the cells were incubated with M13 phages displaying different scFv antibo dies against cell surface epitopes (A-549 cells), the linking moiety was mouse anti.gp8 antibodies. These antibodies are binding to the M13 phage gp8 proteins on the surface of the phage. After adding primary and/or secondary antibodies and washing the cells twice in 3 % PBS/BSA, paramagnetic Dynalbeads M-450 beads (500,000) coated with Pan Mouse IgG were added (Dynal Biotech, Oslo) and incubated on a plate shaker (400 rpm, 4° C) for 20-30 minutes. The ligands/cells having an affinity member on its surface were captured on Dynalbeads using magnetic isolation and non-binding ligands were removed with 3 x washes in PBS/BSA. Proteinase K lysis buffer (30 µl) was introduced to cell-bead rosettes (made of 1 X Dynazyme II PCR reaction buffer (10 mM Tris-HCl pH 8.8, 1.5 mM MgCl₂, 50 mM KCl and 0.1 5 Triton X-100) added 0.45 % (v/v) NP-40, 0.45 % (v/v) Tween 20 and 200 µg/ml Proteinase K. The samples were incubated at 55° C for at least 60 minutes, but typically over night at 55° C, in a sealed (Eppendorf 'Pierce it lite' foil) 96 -well PCR plate on Eppendorf Mastercycler Gradient PCR machine followed by 30 minutes at 95° C to inactivate the enzyme.

To detect the ligands via detection of the PM-1 cells, PCR was applied. The primers for amplifying a short region (∼400 bp) of the household β-actingene inside eukaryotic cells were purchased from MWG Biotech: β-act sense, caagagatggccacggctgct, and β-act antisense, tccttctgcatcctgtcggca. Dynazyme II polymerase (0.8 U per reaction, Finnzymes) was used together with its 10 x buffer. Typically, 5 µl of template (proteinase K treated cells) was applied per reaction using 200 µM dNTP mix (Fermentas) and 77 µM primers in a 30 µl reaction. A standard Taq polymerase PCR program was run using a PCR machine (mentioned above'): 94° C for 5 minutes initially, then 35 cycles of 94° C (1 min), 66° C (1 min) and 72° C (1 min), ending with 72° C (5 min) and 4°C (store). The products were analysed using electrophoresis of samples in a 2 % agarose gel.

### Results/Discussion:

PM-1 cells were either coated with Moc-31 antibodies or anti human HLA-ABC. Figure 1 shows PM-1 cells coated with different amount of Moc31 IgG antibodies. There is a signal (PCR band at 400 bp) even down to 0.1 ng of IgG added to cells. This is corresponding to 40 pg of a scFv antibody or 50 µl from an E.coli expression culture at a concentration of 0.8 µg/L. Again, this equals a density of 16,000 Moc31 molecules per PM-1 cell. Recording data from the related HLA-ABC experiment gave the same results (results not shown). These experiments clearly shows that it is possible to coat cells with antibodies in order to use PCR to indirectly detect the IgG linking moiety (simulating the affinity member). Only if there is a link between the cells and the beads it is possible to isolate cells and use PCR to detect the presence of them.

Another experiment was also performed in order to test if it was possible to coat eukaryotic PM-1 cells with antibodies in bacterial expression medium. To use bacterial expression medium it had to be made isotonic with sterile water. (Isotonic PBS contains 138 mM NaCl while LB Broth bacteria expression medium has a higher concentration of NaCl (171 mM)). The LB expression/medium tested for PM-1 cells also contained 100 µM IPTG, 100 µg/ml ampillicilin and 2 mg/ml lysozyme and before adding to PM-1 cells it was added 0.3 (w/v) % BSA (final). The results of the PCR product of enriched PM-1 cells analysed on gel clearly suggest that it is possible to use isotonic LB to coat eukaryotic cells (Figure 2). This is important because it makes it possible to screen a large number of bacterial clones directly from expression plates. Note that the level of β-actin PCR product for the negative control is higher than in Figure 1 making it difficult to get a better detection than 1 ng in this experiment.

Similar results as shown for the mouse IgG antibodies above were also shown in capturing eukaryotic cells coated with scFv antibodies having c-myc and His₆-tags (results not shown here, but is illustrated in example 2). Coating cells with scFv antibodies as primary antibodies required however adding a secondary mouse IgG antibody to the cells to bind to the c-myc or His tags. This made it possible to still use magnetic beads coated with pan mouse IgG in capturing coated cells.

In addition, it was also possible to coat eukaryotic cells with M13 phages displaying different scFv antibodies against cell surface epitopes (A-549 cells). In this experiments the A-549 cells were first covered with M13 phages, then washed and added mouse anti gp8 antibodies. These antibodies are binding to the M13 phage gp8 proteins on the surface of the phage. The A-549-phage-antibody complexes were washed again and A-549 cells were captured via the phage-antibody linkage with Dynabeads M-450 Pan Mouse IgG. Again the detection is by PCR of the β-actin gene inside the A-549 cells (Figure 3). Only if the link (phage-gp8 antibody-Pan mouse IgG) between the beads and cells is formed it is possible to detect A-549 cells by PCR The results indicated that most of the M13 phage samples with displayed scFvs that were tested bind to A. 549 cells (15 of 16 samples).

To conclude, the method to indirectly detect potential library members such as IgG antibodies, scFv antibodies and the bacteriophage M13 with attached scFvs, via detecting the ligand they bind to, was successful. Because of the detection step is on the ligand molecule or at least an entity directly associated with the ligand molecule), it should now be possible to not only to detect biological libraries but also chemical libraries.

### Example 2:

### Lowering the number of cells that can be used for ligand - optimisation.

### Abstract:

Is it possible to indirectly detect scFv antibodies binding to cell surface epitopes by lowering the number of cells (ligands) to 5,000 per reaction? Theoretically, if the background is eliminated even single cell PCR amplification of ligand DNA may be applied, but this may be requiring performing two different PCR runs.

### Materials and Methods:

### See Example 1.

Generally, 1,000 - 100,000 cells per ELISA well were tested in a 96 U-plate. 5 µl of expressed scFv diluted into 50 µl coating volume (3 % BSA in PBS) was applied. The number of beads was kept as ***5:1*** for 10,000-100,000 cells, but was increased to 10:1 for 1,000 to 5,000 cells.

### Results/Discussion:

It is important to lower the number of cells expressing ligand that can used for a AffiSelection procedure. To grow eukaryotic cells in culture is time consuming and requires a lot of extra resources. To find a minimum amount of A-549 cancer cells that can be used for this technique, the cell number was varied from 100,000 down to 1,000. Figure 4 suggests that it is possible to lower the number of cells expressing ligand down to 5,000 and still detect enriched cells coated with scFvs.

This implies that without any extra PCR optimisation (for single cell), it is still possible to detect 5,000 cells with just one PCR run making the procedure quite cost efficient.

### Example 3:

### Comparing detection level of ordinary cell ELISA with AffiSelect.

### Abstract:

The Affiselect procedure offered a much better sensitivity than ordinary cell ELISA. Several scFv members that were not detected with ordinary cell ELISA were identified by AffiSelect to bind A-549 cells.

### Materials and Methods:

### See Example 1 for details on AffiSelect.

Cell- ELISA was performed using a standard protocol on A-549 cells: A 96-well microtitre plate was blocked in PBS/3 % BSA at 4 ° C overnight Cells (200,000) were added to each well. The cells were resuspended and washed in PBS/3 % BSA (3 times), incubated with primary antibodies or antibody HLA ABC (MHC class I) IgG and incubated for 60 minutes at 4°C on a plate shaker-(280 rpm), washed in PBS/3 %BSA, and added anti-cmyc (1:4000) and incubated for another 60 minutes at 4° C (280 rpm). After washing in PBS/3 %BSA (3 times 100 µl at 4° C) the cells were resuspended in anti mouse IgG-HRP diluted in 3 % BSA in PBS for 60 minutes at 4° C (280 rpm). Finally, after washing again as described above, TMB substrate solution was added (100 µl) and incubated for 15 minutes at room temperature. The supernatant (75 µl) was removed and analysed at 450 nm.

### Results/Discussion:

Figure 5 is comparing signals from cell ELISA with AffiSelect. The main difference is that the cell ELISA is detecting affinity members on the ligand surface by direct methods, but AffiSelect is detecting exclusively the ligand (the cell) after an affinity purification step. It was necessary to use more cells for the cell ELISA (200,000) than AffiSelect (100,000) to get a good enough signal above background. Still, the AffiSelect signal is stronger and better.

### Example 4:

### Simulating the screening of a small library - minute amount of scFv antibodies expressed in bacteria can be detected via binding to A-549 cells.

### Abstract:

This example is illustrating the selection and detection process for diluted samples. Eukaryotic A-549 cells were harvested and incubated with a spiked positive control scFv in a ratio of 1:384 of positive:negative control scFv. Different bead:cell ratios were used, and for a 20:1 or a 30:1 ratio, it was possible to get a good PCR signal ofDNA for the β-actin gene, corresponding to a large numberof A-549 cells, and implying again that scFvs were binding to cell surface epitopes.

### Materials and Methods:

See Example 1. However, the difference between the method described in Example 1 and in this example 4, is that it now allows screening of a larger volume. This implies using 10 ml plastic tubes instead of 96 U-well plates, 1 ml incubation volume (cells + mabs/scFv antibodies) and 0.5 to 1 ml capture volume (cells + beads), and by mixing at 4° for 1 hour (30 minutes for capture) by slow tilting and rotation (rock'n'roller) at all incubations. Also, a larger washing volume was used (1 to 2.5 ml) at all steps. The larger incubation volume facilitates screening several more clones by pooling samples. The number of A-549 cells (ligand) per tube was kept constant as 100,000, and different amounts of beads were added (from a 10-fold excess to 30-fold excess of beads).

### Results and discussion:

A scFv antibody, scFvA, binding to a surface marker of A-549 cells was diluted in a non-binding scFv antibody PhOx at a ratio of 1:384 and incubated with A-549 cells. This ratio corresponds to the chance of picking one positive clone from one among 3 83 negative wells of a 384-plate. This ratio is important in order to simulate a screening of a large number of clones (40,000 clones = 105 x 384-well plates) with a low frequency of positive hits (e.g. 1 per 1,000 or 10,000). Instead of screening individual wells, the method of purifying the ligand allows pooling all wells of a 384-plate into one single tube and a performing a fast screen of several clones at the same time. Antibodies with no affinity towards the ligands were removed by washing (as described in previous examples), and only if there is a positive antibody binding to the ligand, a link between the ligand and bead is formed which facilitates purification.

Different ratios of bead:cells were used. It was not possible to indirectly detect scFvs binding to cells with a 10:1 ratio (Figure 6, spike 0.5 and 1 ml). However, for the ratios 20:1 and 30:1 there was a good PCR signal for spiked samples. The similar background binding as recorded for the negative control scFv PhOx was much lower. All the positive screening controls (undiluted scFv A, MHC, and pos PCR control (+)) were ok, in addition the negative screening controls LB and BSA (Figure 6). This suggests that a good PCR signal can be found for even diluted scFv antibodies. This is also in accordance with the results shown in example 1.

The detection limit of the scFv antibodies relative to the number of epitopes on the cancer cells surface can be compared using Table 1. Even testing only 50 µl from an expression culture containing a very low scFv protein concentration, a molar excess of 200:1 of scFv to a low expressing surface epitope (1,000 epitopes per cell) of 50,000 cancer cell is still possible. This method should therefore be very well suited for isolating library members on low expressed ligand epitopes (lower that 1,000) molecules. Since the detection may potentially be performed on just one single cell, the capacity to pick up such clones should be ideal.

**TABLE 1: Comparing the ratio of expressed scFv (in mg or µg/liter) relative to the number of eukaryote cell epitopes available for coating by the scFv**

| **Cell epitope\ scFv** | **20 mg/liter** | **1 mg/liter** | **100 µg/liter** | **1 µg/liter** |
|---|---|---|---|---|
| **10 per cell** | **4.0 x 10⁷: 1** | | | |
| **1,000 per cell** | **400,000 : 1** | **20,000 : 1** | **2,000 : 1** | **200 : 1** |
| **10,000** | **40,000 : 1** | **2000 : 1** | **200 : 1** | **20 : 1** |
| **100,000** | **4,000 : 1** | **200 : 1** | **20 : 1** | **2: 1** |
| **500,000** | **1600 : 1** | | | |

| | | | | |
|---|---|---|---|---|
| **50 µl of a scFv expression culture is added to 50,000 cancer cells (Mw scFv = 30,000)** | | | | |

### Example 5:

### Using AffiSelect to pick target scFv clones from a phage display enriched human antibody library against a lung carcinoma cell line.

### Abstract:

A human antibody phage library was panned against potential surface antigens on a lung carcinoma cell line with our in-house C.B.A.S. panning technology. Individual scFv clones against the cancer cell line were identified from an isolated polyclonal mixture ('library') of phage display enriched scFv clones comparing PCR patterns of the carcinoma cell line with the two negative cell types HUVEC and PBL. The positive clones identified were verified using FACS. This illustrates that AffiSelect can be used to screen enriched antibody libraries to identify individual clones binding to target cell surface epitopes.

### Materials and Methods:

### Cells

The lung carcinoma cell line (A-549, ATCC CCL-185; kindly provided by Viventia Inc.) and the endothelial cell line (HUVEC, ATCC CRL-1730; kindly provided by Viventia Inc.) were used for panning and screening experiments. A-549 was cultured in Ham's F12 (BioWhittaker) supplemented with 10% FCS and 2mM glutamine, and likewise the endothelial cell line in RPMI-1640 medium supplemented with 10% FCS, 15 mg/l endothelial growth factor supplement (Sigma) and 50 mg/l heparin (Sigma). Cultivations were at 37°C maintaining a 5% CO₂ and humidified atmosphere. Cells were harvested in lmM EDTA in PBS adding washing steps in PB S before and after, and they were counted in a Bürker Haemocytometer. Peripheral blood lymphocytes (PBL) were isolated from fresh human blood or buffy coats with lymphoprep (Axis-Shield). Cells were either resuspended in PBS added 3% BSA (Sigma) or added 4 % skimmed milk (Merck).

### Lectin-coated magnetic beads

Lectin (WGA: Wheat Germ, *Triticum vulgaris;* Calbiochem) was immobilised on M-450 epoxy beads (Dynal) according to manufacturer's protocol. The Dynal beads were washed and incubated in 0.1M borate buffer, pH 8.5 with 10µg lectin/10⁷ beads slow tilting and rotating overnight at room temperature. Then they were washed three times in PBS/0.1% BSA and stored at 4°C in PBS/0.1% BSA/0.02% sodium azide at 4x10⁸ beads/ml.

### Phage scFv display enrichment.

An IgM scFv library constructed from PBL of 6 healthy donors and cloned into a phagemid display system based on pSEX 81 (Welschof et al., 1997, PNAS 94:1902-1907, Løset, et al., 2005, J. Immunol. Methods 299:47-62) was used in panning experiments.

Phages binding to common cell proteins were removed by incubating 3.75 x 10¹² phages *(150 µl library of 2.5x10¹³ cfu*/*ml)* with 2x10⁵-2x10⁶/ml non-tumour cells in a pre-blocked tube (3 % BSA or 4% milk in PBS), rotating for 1 hr at 4°C (= negative pre-panning).

Two negative pre-panning steps were performed one after the other. At first, the library was incubated with PBL cells, then washing the PBL twice with block solution after panning. Next, this pre-panned phage library plus the two wash-supernatants were incubated with HUVEC cells in a pre-blocked tube rotating for 1-2 hrs at 4°C. After two washes of the second negative pre-panning, the supernatant plus wash-supernatants were incubated with target lung tumour cells as described above (panning).

To separate free phages from cell-bound phages, the following washing procedures were followed. Either by a modified organic phase separation according to the BRASIL method (Giordano et al., 2001, Nature Med. 11; 1249-1253) including one or two washing steps before organic phase separation, or by lectin coated magnetic beads. For the modified BRASIL method, 2x150µl of the tumour cell suspension after panning were processed with one or two washing steps in 1 ml PBS/0.4% BSA before resuspending them again in 300µl PBS/0.4% BSA. These cells (2 x 150 µl) were then layered on top of 2 x 300µl organic phase solution (dibutyl phthalate:cyclohexane 9:1 [v:v]; d=1.03 g ml⁻¹) and centrifuged at 10,000 x g for 10 min at room temperature (RT). The tubes were snap frozen in liquid nitrogen, the bottom of the tube was sliced off and the cell pellet was transferred to another (blocked) tube. For the magnetic bead washing procedure, the tumour cells after panning were spun down (500 x g, 5 min. 4°C), resuspended in PBS/0.1% BSA at 5x10⁶ cells/ml and incubated with lectin-coated beads (1:4 cell:bead ratio) rotating for 20 minutes at 4°C. The beads were washed 10 times in 1 ml PBS/0.1% BSA using a magnet and resuspended in 200µl PBS/0.1% BSA.

For infection, cell pellets or beads were added to 10 ml E.Coli XL1 Blue in log phase and incubated at 37°C for 15 min. at 60 rpm, then for 45 min at 200 rpm. Bacteria were plated at different concentrations on small LB-TAG plates (LB added 100 µg/ml ampilicillin, 30 µg/ml tetracyclin, 100 mM glucose) for evaluation, and on two similar large rectangular plates (243x243x18 mm; Nunc) for later packaging. All plates were incubated overnight at 37°C.

Bacteria from the two large plates were harvested by scraping. The bacteria were then propagated in liquid culture, and the phagemid in those cells was packaged into phage particles with M13 helper phage (Stratagene), overnight at 30°C. The next day, phages were precipitated with PEG/NaCl and resuspended in 1 ml PBS of which 100µl phages (approx. 10¹³ cfu) were used for in a new round of panning.

### Polyclonal phage ELISA

Tumour and PBL cells were incubated for 1 hr at 4°C with 100µl phages (1:10-1:10⁶ dilutions) from each panning round in a pre-blocked (PBS/3% BSA) ELISA well. Then, the cells were incubated for 1 hr at 4°C with 100µl rabbit anti-Fd (Sigma; 1:4000) antibody. The ELISA was developed in horseradish peroxidase (HRP) conjugated goat-anti-rabbit IgG antibody (Dako; 1:4000) and the substrate ABTS (Calbiochem). The absorption was measured at 405 nm. Between each incubation step the wells were washed 3 times in PBS.

### Expression and purification of scFv in E.Coli

Panning rounds showing an increase in tumour-specific binding (polyclonal phage ELISA) were candidates for cloning into the secretion vector pHOG21 (Kipriyanov et al., 1997, J. Immunol. Methods, 200: 67-77). The polyclonal.collection of scFv DNA was transformed into competent XL-1 Blue *E*.*Coli* cells and plated on LB-TAG 22 x 22 cm bioassay trays (Corning). Individual clones were picked using QPix2 robot (Genetix, ltd., UK) into 384-well plates containing LB-TAGgrowth medium [100 µg/ml ampilicillin (A), 30 µg/ml tetracycline (T), 1 % (w/v) glucose (G)] added - 4 % glycerol and grown over night at 37° C. LB-TAG medium for expression was seeded from 384-well plates [LB-TAG w/glycerol] and expressed as described previously (Dörsam et al., 1997, FEBS Letters, 414: 7-13) or in 96 deep well format (100-200 microliter volume). Expressions were performed overnight at 30°C in LB-TA with 100 µM IPTG (Sigma). The bacteria cell suspension was added PBS/3. % BSA and were stored frozen at -20° C. By thawing them again they were centrifuged for 10 min. at 4000 rpm, 4°C, and the supernatants (containing the expressed scFv) were used in further experiments.

### AffSelect method

### See Example 1.

However, in this experiments 5µl aliquots of 96 deep well scFv expressions were tested on tumour cells (A-549), PBL and HUVEC using the AffiSelect method. For this, 2.2x10⁴ cells were pelleted (1600 rpm, 4°C, 5 min.) and incubated shaking for 1 hour at 4°C together with 5µl expression sample and 45µl PBS/3% BSA. The cells were washed with 150µl PBS/3% BSA and centrifuged (1600 rpm, 4°C, 5 min.). After the adding 50µl anti-c-myc antibody (Invitrogen; 1:500) to the pellets, the plates were incubated shaking for another 1 hour at 4°C.

The cells were washed twice as described before and incubated shaking for 1 hour at 4°C together with 25µl M-450 pan mouse IgG bead (Dynal) suspension of 5x10⁶ beads/ml (cell:bead ratio =1:5). After washing the beads twice with 100µl PBS/3% BSA on a magnet, they were resuspended in 100µl PBS and transferred to a PCR plate.

The bead samples were incubated overnight at 55°C in 30µl PCR buffer containing proteinase K (200µg/ml), 0.45% Tween 20 and 0.45% NP40, and stored afterwards at -20°C until use. After heat-inactivating the proteinase K for 30 min. at 95°C, the samples were placed on a magnet and 5µl of the supernatant was used for PCR with human beta-actin specific primers.

PCR conditions as described in example 1.

### FACS

1-5x10⁵ cells were stained for I hour at 4°C with 50µl scFv deep well expression. After washing 3 times with 150µl PBS and centrifugation (1600 rpm, 5 min, 4°C), the cells were incubated with 50µl FITC-conjugated anti-c-myc antibody (Invitrogen; 1:30) for 1 hour at 4°C. Cells were washed 3 times as described above, fixed with 200µl PBS/1-2% formalin (Sigma) and stored at 4°C until being measured with a FACS machine(Coulter).

### Results/Discussion:

Results of the polyclonal phage ELISA revealed that after only 2 rounds of panning an increase in phages binding to the tumour cells was seen with both methods of washing (beads or modified BASIL). In contrast, by using the modified BRASIL method an increased enrichment (seen after diluting the phages) after the third and fourth round of panning was obtained when compared to bead method (Figure 10).

After two washes and organic phase centrifugation, scFv expressions from round 3 of panning were tested for its specificity in AffiSelecT against PBL, HUVEC and the tumor cell line A-549 (Figure 11). As shown in the example of figure 11, eight scFv expression samples tested against the tumour cell line showed PCR-amplified beta-actin DNA, whereas no such product was seen with HUVEC and PBL. This shows that in this case 8 of the tested scFv samples were binding to the tumor cell line, but not to HUVEC or PBL.

After confirming tumor cell line-specific specific binding by FACS measurements (Figure 12 shows an exemplary clone), ten unique and tumor cell line-specific clones were found.

This shows that AffiSelect is a sensitive method of picking candidates from a small antibody library. The positive clones identified by AffiSelect verified by FACS confirm that this method of selection is very well suited for picking scFv candidates binding to cell surface epitopes.

## Claims

1. A method of screening a library of molecules to identify and/or select one or more members thereof which are candidate binding partners for one or more ligands comprising:
a) contacting an expression library in solution with one or more ligands wherein said expression library is not attached to a solid phase;
b) capturing ligands which have become bound to one or more members of the expression library onto a solid phase, wherein said capture onto a solid phase step is facilitated by the expression library members and wherein said capture onto a solid phase involves the interaction of a capture molecule on the solid phase with a partner molecule or tag associated with the members of the expression library; and
c) detecting the presence of a ligand, thereby detecting the presence of one or more members of the expression library which are candidate binding partners for the ligand.

2. The method of claim 1 wherein said expression library is a phage display library or a bacterial expression library.

3. The method of claim 1 or claim 2 wherein said expression library is an antibody expression library.

4. The method of claim 3 wherein said antibody expression library comprises scFv antibodies.

5. The method of any one of claims I to 4 wherein said ligand is a cell surface molecule or is attached to a solid phase.

6. The method of claim 5 wherein said cell surface molecule is provided as a component of whole cells or a membrane fraction of cells.

7. The method of claim 5 or claim 6 wherein said cells are eukaryotic cells.

8. The method of any one of claims 5 to 7 wherein said cells are associated with a disease state.

9. The method of any one of claims 5 to 8 wherein said cells are cancer cells.

10. The method of any one of claims 5 to 9 wherein said cells arc transformed or transfected with nucleic acid encoding said ligand or are transformed or transfected with nucleic acids encoding a library of ligands, or are cells which overexpress said ligand.

11. The method of claim 10 wherein said library is derived from a tumour cell or a virus cell.

12. The method of claim 5 wherein said ligand attached to a solid phase is associated with the nucleic acid encoding it or is associated with a molecular tag.

13. The method of claim 5 or claim 12 wherein said solid phase to which said ligand is attached is particulate and non magnetic.

14. The method of any one of claims 1 to 13 wherein said ligand contains or is associated with a reporter moiety to enable detection of the ligand.

15. The method of claim 14 wherein said reporter moiety is a DNA molecule.

16. The method of claim 14 or claim 15 wherein said ligand is a cell surface molecule and said reporter moiety is a DNA molecule present in the cells on which the ligand is expressed.

17. The method of claim 15 or claim 16 wherein said DNA molecule is an endogenous housekeeping gene or is an exogenous reporter moiety.

18. The method of any one of claims 1 to 17 wherein more than one ligand is provided in step (a).

19. The method of any one of claims 1 to 18 wherein said contacting step is carried out in the presence of bacterial expression medium.

20. The method of any one of claims 5 to 11 or 14 to 19 wherein less than 5000 or 1000 cells are provided.

21. The method of any one of claims 1 to 20 wherein said solid phase in step (b) is magnetic and preferably particulate.

22. The method of any one of claims 1 to 21 wherein said detection step is carried out by detecting the presence of a reporter moiety on or associated with the ligand.

23. The method of claim 22 wherein the detection step is by PCR.

24. The method of any one of claims 1 to 23 wherein multiple members of the expression library are present in the same assay compartment and then brought into contact with one or more ligands.

25. The method of any one of claims 1 to 24 wherein said method further comprises one or more panning steps in which the complexity of the expression library to be used in step (a) is reduced by panning the library with the target ligand(s).

26. The method of claim 25 wherein said panning step involves (i) contacting an expression library with the target ligand(s), (ii) subjecting the target ligand(s) to at least one washing step, and (iii) separation of the target ligand(s) which have become bound to expression library members from unbound members of the expression library by separation through an organic phase, thereby separating candidate binding partners for said target ligand(s) from other library members.

27. The method of any one of claims 1 to 26 wherein said candidate binding partners or the ligands are subjected to further analysis.

28. The method of any one of claims 1 to 27 further comprising a step wherein said candidate binding partners are expressed or produced in isolation from said ligands.

29. A method for isolating and/or identifying an unknown ligand comprising the steps as defined in any one of claims 1 to 28, and further comprising (d) isolating one or more expression library members which bind to said unknown ligand and (e) using said library member to isolate and/or identify the ligand to which it binds.

30. A method of selecting, identifying and/or isolating a library member which is a specific binding partner for a ligand, or a method of selecting, identifying and/or isolating a ligand, from an expression library, said method comprising the steps as defined in any one of claims I to 28 to select molecules which display certain properties and optionally (e) identifying and/or isolating the relevant library member(s) which are specific binding partners for the ligand and optionally (f) using said library members to identify the ligand to which it binds.

31. The method of claim 29 or claim 30 wherein step (e) of claim 29 or step (f) of claim 30 comprises using said library member to screen a cDNA library prepared from cells on which the unknown ligand is expressed.

32. A method for isolating and/or identifying an unknown ligand comprising:
(a) contacting one or more specific binding partners for the unknown ligand in solution with a library of potential ligands expressed in cells wherein said specific binding partner is not attached to a solid phase;
(b) capturing ligands which have become bound to one or more of the specific binding partners onto a solid phase wherein said capture onto a solid phase step is facilitated by the specific binding partners and wherein said capture onto a solid phase involves the interaction of a capture molecule on the solid phase with a partner molecule or tag associated with the specific binding partners; and
(c) detecting the presence of a ligand, thereby detecting the presence of one or more ligands which are candidate unknown ligands.

33. The method of claim 32 wherein said one or more specific binding partners are identified or selected using a method as defined in any one of claims 1 to 25.

34. The method of claim 32 or claim 33 wherein said library is derived from a cell on which the unknown ligand is expressed.

35. The method of any one of claims 1 to 34 further comprising the step of manufacturing or expressing said identified binding partner or ligand, or a component, fragment, variant, or derivative thereof, and optionally formulating said binding partner or ligand with at least one pharmaceutically acceptable carrier or excipient.

36. A method of screening a library of molecules to identify and/or select one or more members thereof which are candidate binding partners for one or more ligands, comprising:
(i) pooling a number of bacterial clones which are capable of expressing expression library members, or pooling a number of expression library members which have been produced by a number of bacterial clones, in a single assay compartment;
(ii) if a number of bacterial clones are pooled in step (i), inducing the bacterial clones to express said expression library members;
(iii) contacting said expression library members in solution with one or more ligands wherein said expression library is not attached to a solid phase;
(iv) determining positive assay compartments in which one or more expression library members have become bound to ligand wherein said determining step is carried out in accordance with steps (b) and (c) as defined in any one of claims 1 to 23;
(v) carrying out a selective expansion of the clones which are present in the positive assay compartments, and repeating steps (iii) and (iv);
(vi) identifying one or more bacterial clones which express the binding partners for said ligand.

## Patentansprüche

1. Verfahren für das Screenen einer Bibliothek von Molekülen, um ein oder mehrere Mitglieder davon zu identifizieren und/oder zu selektieren, die Kandidaten-Bindungspartner für einen oder mehrere Liganden sind, welches Folgendes umfasst:
a) Kontaktieren einer Expressionsbibliothek in Lösung mit einem oder mehreren Liganden, wobei die Expressionsbibliothek nicht an eine feste Phase angeheftet ist;
b) Einfangen von Liganden, die an ein oder mehrere Mitglieder der Expressionsbibliothek auf eine feste Phase gebunden wurden, wobei der Schritt des Einfangens auf eine feste Phase durch die Expressionsbibliothek-Mitglieder erleichtert wird und wobei das Einfangen auf eine feste Phase die Interaktion eines Einfangmoleküls auf der festen Phase mit einem Partnermolekül oder Tag, das mit den Mitgliedern der Expressionsbibliothek assoziiert ist, beinhaltet; und
c) Nachweisen des Vorhandenseins eines Liganden, wodurch die Anwesenheit von einem oder mehreren Mitgliedern der Expressionsbibliothek detektiert wird, die Kandidaten-Bindungspartner für den Liganden sind.

2. Verfahren von Anspruch 1, wobei die Expressionsbibliothek eine Phagen-Display-Bibliothek oder eine bakterielle Expressionsbibliothek ist.

3. Verfahren von Anspruch 1 oder Anspruch 2, wobei die Expressionsbibliothek eine Antikörper-Expressionsbibliothek ist.

4. Verfahren von Anspruch 3, wobei die Antikörper-Expressionsbibliothek scFv-Antikörper umfasst.

5. Verfahren von einem beliebigen der Ansprüche 1 bis 4, wobei der Ligand ein Zelloberflächenmolekül ist oder an eine feste Phase angeheftet ist.

6. Verfahren von Anspruch 5, wobei das Zelloberflächenmolekül als eine Komponente von Ganzzellen oder eine Membranfraktion von Zellen vorgesehen wird.

7. Verfahren von Anspruch 5 oder Anspruch 6, wobei die Zellen eukaryotische Zellen sind.

8. Verfahren von einem beliebigen der Ansprüche 5 bis 7, wobei die Zellen mit einem Erkrankungszustand assoziiert sind.

9. Verfahren von einem beliebigen der Ansprüche 5 bis 8, wobei die Zellen Krebszellen sind.

10. Verfahren von einem beliebigen der Ansprüche 5 bis 9, wobei die Zellen mit Nukleinsäure, die den Liganden codiert, transformiert oder transfiziert sind oder mit Nukleinsäuren, die eine Bibliothek von Liganden codieren, transformiert oder transfiziert sind, oder Zellen sind, die den Liganden überexprimieren.

11. Verfahren von Anspruch 10, wobei die Bibliothek aus einer Tumorzelle oder einer Viruszelle abgeleitet ist.

12. Verfahren von Anspruch 5, wobei der an eine feste Phase angeheftete Ligand mit der für ihn codierenden Nukleinsäure assoziiert ist oder mit einem molekularen Tag bzw. Anhängsel assoziiert ist.

13. Verfahren von Anspruch 5 oder Anspruch 12, wobei die feste Phase, an welche der Ligand angeheftet ist, partikelförmig und nicht-magnetisch ist.

14. Verfahren von einem beliebigen der Ansprüche 1 bis 13, wobei der Ligand eine Reportereinheit enthält oder damit assoziiert ist, um den Nachweis des Liganden zu ermöglichen.

15. Verfahren von Anspruch 14, wobei die Reportereinheit ein DNA-Molekül ist.

16. Verfahren von Anspruch 14 oder Anspruch 15, wobei der Ligand ein Zelloberflächenmolekül ist und die Reportereinheit ein DNA-Molekül ist, das in den Zellen vorhanden ist, auf welchen der Ligand exprimiert wird.

17. Verfahren von Anspruch 15 oder Anspruch 16, wobei das DNA-Molekül ein endogenes Haushaltsgen ist oder eine exogene Reportereinheit ist.

18. verfahren von einem beliebigen der Ansprüche 1 bis 17, wobei in Schritt (a) mehr als ein Ligand bereitgestellt wird.

19. Verfahren von einem beliebigen der Ansprüche 1 bis 18, wobei der Kontaktierungsschritt in Gegenwart von bakteriellem Expressionsmedium durchgeführt wird.

20. Verfahren von einem beliebigen der Ansprüche 5 bis 11 oder 14 bis 19, wobei weniger als 5000 oder 1000 Zellen bereitgestellt werden.

21. Verfahren von einem beliebigen der Ansprüche 1 bis 20, wobei die feste Phase in Schritt (b) magnetisch und vorzugsweise partikelförmig ist.

22. Verfahren von einem beliebigen der Ansprüche 1 bis 21, wobei der Nachweisschritt durch Nachweisen des Vorhandenseins einer Reportereinheit auf dem Liganden, oder assoziiert mit diesem, durchgeführt wird.

23. verfahren von Anspruch 22, wobei der Nachweisschritt durch PCR erfolgt.

24. Verfahren von einem beliebigen der Ansprüche 1 bis 23, wobei mehrere Mitglieder der Expressionsbibliothek in demselben Assay-Kompartiment vorliegen und dann mit einem oder mehreren Liganden in Kontakt gebracht werden.

25. Verfahren von einem beliebigen der Ansprüche 1 bis 24, wobei das Verfahren ferner einen oder mehrere "Panning"-Schritte umfasst, in denen die Komplexität der Expressionsbibliothek, die in Schritt (a) verwendet werden soll, durch Panning der Bibliothek mit dem/den zielliganden verringert wird.

26. Verfahren von Anspruch 25, wobei der Panning-Schritt (i) das Kontaktieren einer Expressionsbibliothek mit dem/den zielliganden, (ii) das Unterziehen des/der Zielliganden wenigstens einem Waschschritt und (iii) das Abtrennen des/der Zielliganden, der/die an Expressionsbibliothek-Mitglieder gebunden wurde/n, von ungebundenen Mitgliedern der Expressionsbibliothek mittels Trennen durch eine organische Phase, wodurch Kandidaten-Bindungspartner für den/die Zielliganden von anderen Bibliotheksmitgliedern getrennt werden, beinhaltet.

27. Verfahren von einem beliebigen der Ansprüche 1 bis 26, wobei die Kandidaten-Bindungspartner oder die Liganden einer weiteren Analyse unterworfen werden.

28. Verfahren von einem beliebigen der Ansprüche 1 bis 27, das ferner einen Schritt umfasst, in dem die Kandidaten-Bindungspartner in Isolierung von den Liganden exprimiert oder produziert werden.

29. Verfahren zum Isolieren und/oder Identifizieren eines unbekannten Liganden, das die Schritte, wie in einem beliebigen der Ansprüche 1 bis 28 definiert, umfasst und weiterhin (d) das Isolieren von einem oder mehreren Expressionsbibliothek-Mitgliedern, welche an den unbekannten Liganden binden, und (e) das Nutzen des Bibliothekmitglieds zum Isolieren und/oder Identifizieren des Liganden, an welchen dieses bindet, umfasst.

30. Verfahren zum Selektieren, Identifizieren und/oder Isolieren eines Bibliothekmitglieds, bei dem es sich um einen spezifischen Bindungspartner für einen Liganden handelt, oder ein Verfahren zum Selektieren, Identifizieren und/oder Isolieren eines Liganden aus einer Expressionsbibliothek, wobei das Verfahren die Schritte, wie in einem beliebigen der Ansprüche 1 bis 28 definiert, zum Selektieren von Molekülen, welche bestimmte Eigenschaften zeigen, und gegebenenfalls (e) das Identifizieren und/oder Isolieren des/der relevanten Bibliotheksmitglieds/Bibliotheksmitglieder, bei dem/denen es sich um spezifische Bindungspartner für den Liganden handelt, und gegebenenfalls (f) das Nutzen der Bibliotheksmitglieder zum Identifizieren des Liganden, an welchen diese(s) binden bzw. bindet, umfasst.

31. Verfahren von Anspruch 29 oder Anspruch 30, wobei der Schritt (e) von Anspruch 29 oder der Schritt (f) von Anspruch 30 das Nutzen des Bibliothekmitglieds zum Screenen einer cDNA-Bibliothek umfasst, welche aus Zellen hergestellt wurde, auf denen der unbekannte Ligand exprimiert wird.

32. Verfahren zum Isolieren und/oder Identifizieren eines unbekannten Liganden, das Folgendes umfasst:
(a) Kontaktieren von einem oder mehreren spezifischen Bindungspartnern für den unbekannten Liganden in Lösung mit einer Bibliothek von potentiellen Liganden, die in Zellen exprimiert werden, wobei der spezifische Bindungspartner nicht an eine feste Phase angeheftet ist;
(b) Einfangen von Liganden, welche an einen oder mehrere der spezifischen Bindungspartner auf eine feste Phase gebunden worden sind, wobei der Schritt des Einfangens auf eine feste Phase durch die spezifischen Bindungspartner erleichtert wird und wobei das Einfangen auf eine feste Phase die Interaktion eines Einfangmoleküls auf der festen Phase mit einem Partnermolekül oder Tag, das mit den spezifischen Bindungspartnern assoziiert ist, beinhaltet; und
c) Detektieren des Vorhandenseins eines Liganden, wodurch die Anwesenheit von einem oder mehreren Liganden nachgewiesen wird, die unbekannte Kandidaten-Liganden sind.

33. Verfahren von Anspruch 32, wobei der eine oder die mehreren spezifischen Bindungspartner mit Hilfe eines Verfahrens, wie in einem beliebigen der Ansprüche 1 bis 25 definiert, identifiziert oder selektiert werden.

34. Verfahren von Anspruch 32 oder Anspruch 33, wobei die Bibliothek von einer zelle abgeleitet ist, auf welcher der unbekannte Ligand exprimiert wird.

35. Verfahren von einem beliebigen der Ansprüche 1 bis 34, das ferner den Schritt des Herstellens oder Exprimierens des identifizierten Bindungspartners oder Liganden, oder einer Komponente, eines Fragments, einer Variante oder eines Derivats davon, und gegebenenfalls das Formulieren des Bindungspartners oder Liganden mit wenigstens einem pharmazeutisch annehmbaren Träger oder Hilfsstoff umfasst.

36. Verfahren zum Screenen einer Bibliothek von Molekülen zum Identifizieren und/oder Selektieren von einem oder mehreren Mitgliedern davon, welche Kandidaten-Bindungspartner für einen oder mehrere Liganden sind, das Folgendes umfasst:
(i) Poolen einer Anzahl von bakteriellen Klonen, welche zum Exprimieren von Expressionsbibliotheksmitgliedern in der Lage sind, oder Poolen einer Anzahl von Expressionsbibliotheksmitgliedern, die durch eine Anzahl von bakteriellen Klonen produziert wurden, in einem einzelnen Assay-Kompartiment;
(ii) wenn eine Anzahl an bakteriellen Klonen in Schritt (i) gepoolt ist, Induzieren, dass die bakteriellen Klone die Expressionsbibliotheksmitglieder exprimieren;
(iii) Kontaktieren der Expressionsbibliotheksmitglieder in Lösung mit einem oder mehreren Liganden, wobei die Expressionsbibliothek nicht an eine feste Phase angeheftet ist;
(iv) Bestimmen von positiven Assay-Kompartimenten, in welchen ein oder mehrere Expressionsbibliotheksmitglieder an Ligand gebunden worden sind, wobei der Bestimmungsschritt gemäß den Schritten (b) und (c), wie in einem beliebigen der Ansprüche 1 bis 23 definiert, durchgeführt wird;
(v) Durchführen einer selektiven Expansion bzw, Vermehrung der Klone, welche in den positiven Assay-Kompartimenten vorhanden sind, und Wiederholen der Schritte (iii) und (iv);
(vi) Identifizieren von einem oder mehreren bakteriellen Klonen, welche die Bindungspartner für den Liganden exprimieren.

## Revendications

1. Procédé de criblage d'une banque de molécules pour identifier et/ou sélectionner un ou plusieurs membres de celle-ci qui sont des partenaires de liaison candidats pour un ou plusieurs ligands comprenant :
a) la mise en contact d'une banque d'expression en solution avec un ou plusieurs ligands, ladite banque d'expression n'étant pas liée à une phase solide ;
b) la capture de ligands qui sont devenus liés à un ou plusieurs membres de la banque d'expression sur une phase solide, ladite étape de capture sur une phase solide étant facilitée par les membres de la banque d'expression et ladite capture sur une phase solide mettant en oeuvre l'interaction d'une molécule de capture sur la phase solide avec une molécule ou étiquette partenaire associée aux membres de la banque d'expression ; et
c) la détection de la présence d'un ligand, de manière à détecter la présence d'un ou plusieurs membres de la banque d'expression qui sont des partenaires de liaison candidats pour le ligand.

2. Procédé de la revendication 1 dans lequel ladite banque d'expression est une banque de présentation sur phage ou une banque d'expression bactérienne.

3. Procédé de la revendication 1 ou la revendication 2 dans lequel ladite banque d'expression est une banque d'expression d'anticorps.

4. Procédé de la revendication 3 dans lequel ladite banque d'expression d'anticorps comprend des anticorps scFv.

5. Procédé de l'une quelconque des revendications 1 à 4 dans lequel ledit ligand est une molécule de surface cellulaire ou est lié à une phase solide.

6. Procédé de la revendication 5 dans lequel ladite molécule de surface cellulaire est fournie en tant que composant de cellules totales ou fraction de membrane de cellules.

7. Procédé de la revendication 5 ou la revendication 6 dans lequel lesdites cellules sont des cellules eucaryotes.

8. Procédé de l'une quelconque des revendications 5 à 7 dans lequel lesdites cellules sont associées à un état pathologique.

9. Procédé de l'une quelconque des revendications 5 à 8 dans lequel lesdites cellules sont des cellules cancéreuses.

10. Procédé de l'une quelconque des revendications 5 à 9 dans lequel lesdites cellules sont transformées ou transfectées avec un acide nucléique codant pour ledit ligand ou sont transformées ou transfectées avec des acides nucléiques codant pour une banque de ligands, ou sont des cellules qui surexpriment ledit ligand.

11. Procédé de la revendication 10 dans lequel ladite banque est dérivée d'une cellule cancéreuse ou d'une cellule virale.

12. Procédé de la revendication 5 dans lequel ledit ligand lié à une phase solide est associé à l'acide nucléique codant pour celui-ci ou est associé à une étiquette moléculaire.

13. Procédé de la revendication 5 ou la revendication 12 dans lequel ladite phase solide à laquelle ledit ligand est lié est particulaire et non magnétique.

14. Procédé de l'une quelconque des revendications 1 à 13 dans lequel ledit ligand contient ou est associé à un fragment rapporteur pour permettre la détection du ligand.

15. Procédé de la revendication 14 dans lequel ledit fragment rapporteur est une molécule d'ADN.

16. Procédé de la revendication 14 ou la revendication 15 dans lequel ledit ligand est une molécule de surface cellulaire et ledit fragment rapporteur est une molécule d'ADN présente dans les cellules sur lesquelles le ligand est exprimé.

17. Procédé de la revendication 15 ou la revendication 16 dans lequel ladite molécule d'ADN est un gène domestique endogène ou est un fragment rapporteur exogène.

18. Procédé de l'une quelconque des revendications 1 à 17 dans lequel plus d'un ligand est produit dans l'étape (a).

19. Procédé de l'une quelconque des revendications 1 à 18 dans lequel ladite étape de mise en contact est réalisée en présence de milieu d'expression bactérienne.

20. Procédé de l'une quelconque des revendications 5 à 11 ou 14 à 19 dans lequel moins de 5000 ou 1000 cellules sont produites.

21. Procédé de l'une quelconque des revendications 1 à 20 dans lequel ladite phase solide dans l'étape (b) est magnétique et de préférence particulaire.

22. Procédé de l'une quelconque des revendications 1 à 21 dans lequel ladite étape de détection est réalisée en détectant la présence d'un fragment rapporteur sur ou associé au ligand.

23. Procédé de la revendication 22 dans lequel l'étape de détection est réalisée par PCR.

24. Procédé de l'une quelconque des revendications 1 à 23 dans lequel des membres multiples de la banque d'expression sont présents dans le même compartiment d'essai et ensuite mis en contact avec un ou plusieurs ligands.

25. Procédé de l'une quelconque des revendications 1 à 24 dans lequel ledit procédé comprend en outre une ou plusieurs étapes d'adhérence sur plastique dans lesquelles la complexité de la banque d'expression à utiliser dans l'étape (a) est réduite par adhérence sur plastique de la banque avec le(s) ligand(s) cible(s).

26. Procédé de la revendication 25 dans lequel ladite étape d'adhérence sur plastique met en oeuvre (i) la mise en contact d'une banque d'expression avec le(s) ligand(s) cible(s), (ii) la soumission du/des ligand(s) cible(s) à au moins une étape de lavage, et (iii) la séparation du/des ligand(s) cible(s) qui sont devenus liés à des membres de la banque d'expression des membres de la banque d'expression non liés par séparation par l'intermédiaire d'une phase organique, de manière à séparer les partenaires de liaison candidats pour le(s) dit(s) ligand(s) cible(s) des autres membres de la banque.

27. Procédé de l'une quelconque des revendications 1 à 26 dans lequel lesdits partenaires de liaison candidats ou les ligands sont soumis à une analyse supplémentaire.

28. Procédé de l'une quelconque des revendications 1 à 27 comprenant en outre une étape dans laquelle lesdits partenaires de liaison candidats sont exprimés ou produits de façon isolée desdits ligands.

29. Procédé pour isoler et/ou identifier un ligand inconnu comprenant les étapes telles que définies dans l'une quelconque des revendications 1 à 28, et comprenant en outre (d) l'isolement d'un ou plusieurs membres de la banque d'expression qui se lient audit ligand inconnu et (e) l'utilisation dudit membre de la banque pour isoler et/ou identifier le ligand auquel il se lie.

30. Procédé de sélection, identification et/ou isolement d'un membre d'une banque qui est un partenaire de liaison spécifique pour un ligand, ou procédé de sélection, identification et/ou isolement d'un ligand, à partir d'une banque d'expression, ledit procédé comprenant les étapes telles que définies dans l'une quelconque des revendications 1 à 28 pour sélectionner des molécules qui présentent certaines propriétés et facultativement (e) l'identification et/ou l'isolement du/des membre(s) de la banque en question qui sont des partenaires de liaison spécifiques pour le ligand et facultativement (f) l'utilisation desdits membres de la banque pour identifier le ligand auquel il se lie.

31. Procédé de la revendication 29 ou la revendication 30 dans lequel l'étape (e) de la revendication 29 ou l'étape (f) de la revendication 30 comprend l'utilisation dudit membre de la banque pour cribler une banque d'ADNc préparée à partir de cellules sur lesquelles le ligand inconnu est exprimé.

32. Procédé pour isoler et/ou identifier un ligand inconnu comprenant :
(a) la mise en contact d'un ou plusieurs partenaires de liaison spécifiques pour le ligand inconnu en solution avec une banque de ligands potentiels exprimés dans des cellules, ledit partenaire de liaison spécifique n'étant pas lié à une phase solide ;
(b) la capture de ligands qui sont devenus liés à un ou plusieurs des partenaires de liaison spécifiques sur une phase solide, ladite étape de capture sur une phase solide étant facilitée par les partenaires de liaison spécifiques et ladite capture sur une phase solide mettant en oeuvre l'interaction d'une molécule de capture sur la phase solide avec une molécule ou étiquette partenaire associée aux partenaires de liaison spécifiques ; et
(c) la détection de la présence d'un ligand, de manière à détecter la présence d'un ou plusieurs ligands qui sont des ligands inconnus candidats.

33. Procédé de la revendication 32 dans lequel lesdits un ou plusieurs partenaires de liaison spécifiques sont identifiés ou sélectionnés en utilisant un procédé tel que défini dans l'une quelconque des revendications 1 à 25.

34. Procédé de la revendication 32 ou la revendication 33 dans lequel ladite banque est dérivée d'une cellule sur laquelle le ligand inconnu est exprimé.

35. Procédé de l'une quelconque des revendications 1 à 34 comprenant en outre l'étape de fabrication ou d'expression dudit partenaire de liaison ou ligand identifié, ou un composant, fragment, variant, ou dérivé de celui-ci, et facultativement la formulation dudit partenaire de liaison ou ligand avec au moins un véhicule ou excipient pharmaceutiquement acceptable.

36. Procédé de criblage d'une banque de molécules pour identifier et/ou sélectionner un ou plusieurs membres de celle-ci qui sont des partenaires de liaison candidats pour un ou plusieurs ligands, comprenant :
(i) le groupage d'une pluralité de clones bactériens qui sont capables d'exprimer des membres de la banque d'expression, ou le groupage d'une pluralité de membres de la banque d'expression qui ont été produits par une pluralité de clones bactériens, dans un compartiment d'essai unique ;
(ii) si une pluralité de clones bactériens sont groupés dans l'étape (i), l'induction des clones bactériens pour exprimer lesdits membres de la banque d'expression ;
(iii) la mise en contact desdits membres de la banque d'expression avec un ou plusieurs ligands, ladite banque d'expression n'étant pas liée à une phase solide ;
(iv) la détermination des compartiments d'essai positifs dans lesquels un ou plusieurs membres de la banque d'expression sont devenus liés à un ligand, ladite étape de détermination étant réalisée selon les étapes (b) et (c) telles que définies dans l'une quelconque des revendications 1 à 23 ;
(v) la réalisation d'une expansion sélective des clones qui sont présents dans les compartiments d'essai positifs, et la répétition des étapes (iii) et (iv) ;
(vi) l'identification d'un ou plusieurs clones bactériens qui expriment les partenaires de liaison pour ledit ligand.
